# EUROPEAN PATENT SPECIFICATION

(11) **EP 1 545 519 B1**
(45) Date of publication and mention of the grant of the patent: **23.03.2011**
(21) Application number: 03792573.2
(22) Date of filing: 07.08.2003
(51) Int. Cl.: A61K 31/425, A61P 35/00, A61K 31/4745, A61K 31/505, A61K 31/282, A61K 31/337, A61K 31/70

(54) **COMBINATION THERAPY FOR HYPERPROLIFERATIVE DISEASES**
KOMBINATIONSTHERAPIE GEGEN HYPERPROLIFERATIVE ERKRANKUNGEN
THERAPIE DE COMBINAISON POUR MALADIES HYPERPROLIFERATIVES

(30) Priority: 19.08.2002 US 404461 P
(43) Date of publication of application: 29.06.2005
(73) Proprietor: Pfizer Inc., New York, N.Y. 10017-5755 (US); OSI Pharmaceuticals, Inc., Melville, NY 11747 (US)
(72) Inventor: BEEBE, Jean, Saccuzzo, Salem Connecticut 06420 (US); FERRANTE, Karen, Jean, Rhode Island 02818 (US); JANI, Jitesh, Pranlal, Connecticut 06333 (US); SCHAEFFER, Tracey, Lee, Connecticut 06254 (US); HEALEY, Diane, Ingeborg, Connecticut 06443 (US); O'LEARY, James, John, Mystic, Connecticut 06355on, CT 06355 (US)
(74) Representative: Teuten, Andrew John
(86) International application number: PCT/IB2003/003550
(87) International publication number: WO 2004/017964

(56) References cited:
- US-B1- 6 235 764

## Description

### Background of the Invention

This invention relates to the use of a combination of compounds in the preparation of an agent for the treatment of hyperproliferative diseases. More particularly, the present invention relates to the use of (i) a therapeutically effective amount of gemcitabine hydrochloride and (ii) an isothiazole derivative in the preparation of an agent for simultaneous or sequential administration for treating hyperproliferative diseases, such as cancer This invention also relates to pharmaceutical compositions useful in the treatment of hyperproliferative diseases in mammals, containing gemcitabine hydrochloride in combination with an isothiazole derivative, according to formula 1.

Cancer continues to be one of the leading causes of death in the United States and other developed countries. A large number of drugs are currently being tested in clinical trials for the treatment of a wide variety of cancers. One of the preferred approaches to the treatment of cancer has been combination therapy. One of the advantages of combination therapy has been the ability to attack the cancer using agents that have different mechanisms of action. This has been found in some cases to lead to an enhanced efficacy in trials as indicated by improved disease free survival and overall survival from the use of combination protocols.

One of the newer treatments that is been developed is that of target therapy to treat cancer. It is known that a cell may become cancerous by virtue of the transformation of a portion of its DNA into an oncogene (i.e. a gene that upon activation leads to the formation of malignant tumor cells). Many oncogenes encode proteins which are aberrant tyrosine kinases capable of causing cell transformation. Alternatively, the overexpression of a normal proto-oncogenic tyrosine kinase may also result in proliferative disorders, sometimes resulting in a malignant phenotype. It has been shown that certain tyrosine kinases may be mutated or overexpressed in many human cancers such as brain, lung, squamous cell, bladder, gastric, breast, head and neck, oesophageal, gynecological and thyroid cancers. Furthermore, the overexpression of a ligand for a tyrosine kinase receptor may result in an increase in the activation state of the receptor, resulting in proliferation of the tumor cells or endothelial cells. Thus, it is believed that inhibitors of receptor tyrosine kinases, such as the compounds of the present invention, are useful as selective inhibitors of the growth of mammalian cancer cells.

It is known that polypeptide growth factors, such as vascular endothelial growth factor (VEGF) having a high affinity to the human kinase insert-domain-containing receptor (KDR) or the murine fetal liver kinase 1 (FLK-1) receptor, have been associated with the proliferation of endothelial cells and more particularly vasculogenesis and angiogenesis. See PCT international application publication number WO 95/21613 (published August 17, 1995). Agents, such as the compounds of the present invention, that are capable of binding to or modulating the KDR/FLK-1 receptor may be used to treat disorders related to vasculogenesis or angiogenesis such as diabetes, diabetic retinopathy, hemangioma, glioma, melanoma, Kaposi's sarcoma and ovarian, breast, lung, pancreatic, prostate, colon and epidermoid cancer.

### Summary of the Invention

The present invention relates to the use of (i) a therapeutically effective amound of gemcitabine hydrochloride; and (ii) a therapeutically effective amount of a compound of the formula 1 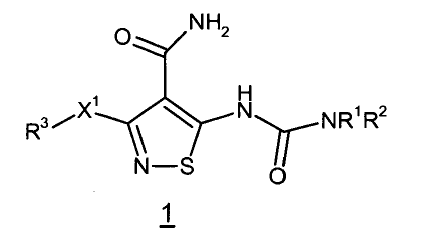 or a pharmaceutically acceptable salt or solvate thereof, wherein:
wherein X¹ is O or S;
R¹ is H, C₁-C₁₀ alkyl, C₂-C₁₀ alkenyl, C₂-C₁₀ alkynyl, -C(O)(C₁-C₁₀ alkyl), -(CH₂)ₜ(C₆-C₁₀ aryl), -(CH₂)ₜ(4-10 membered heterocyclic), -C(O)(CH₂)ₜ(C₆-C₁₀ aryl), or -C(O)(CH₂)ₜ(5-10 membered heterocyclic), wherein t is an integer from 0 to 5; said alkyl group optionally includes 1 or 2 hetero moieties selected from O, S and -N(R⁶)- with the proviso that two O atoms, two S atoms, or an O and S atom are not attached directly to each other; said aryl and heterocyclic R¹ groups are optionally fused to a C₆-C₁₀ aryl group, a C₅-C₈ saturated cyclic group, or a 5-10 membered heterocyclic group; 1 or 2 carbon atoms in the foregoing heterocyclic moieties are optionally substituted by an oxo (=O) moiety; the -(CH₂)ₜ- moieties of the foregoing R¹ groups optionally include a carbon-carbon double or triple bond where t is an integer from 2 to 5; and the foregoing R¹ groups, except H, are optionally substituted by 1 to 3 R⁴ groups;
R² is selected from the list of substituents provided in the definition of R¹, -SO₂(CH₂)ₜ(C₆-C₁₀ aryl), -SO₂(CH₂)ₜ(5-10 membered heterocyclic), and -OR⁵, t is an integer ranging from 0 to 5, the -(CH₂)ₜ- moieties of the foregoing R² groups optionally include a carbon-carbon double or triple bond where t is an integer from 2 to 5, and the foregoing R² groups are optionally substituted by 1 to 3 R⁴ groups;
or R¹ and R² may be taken together with the nitrogen to which each is attached to form a 4-10 membered saturated monocyclic or polycyclic ring or a 5-10 membered heteroaryl ring, wherein said saturated and heteroaryl rings optionally include 1 or 2 heteroatoms selected from O, S and -N(R⁶)- in addition to the nitrogen to which R¹ and R² are attached, said -N(R⁶)- is optionally =N- or -N= where R¹ and R² are taken together as said heteroaryl group, said saturated ring optionally may be partially unsaturated by including 1 or 2 carbon-carbon double bonds, and said saturated and heteroaryl rings, including the R⁶ group of said -N(R⁶)-, are optionally substituted by 1 to 3 R⁴ groups;
R³ is H, C₁-C₁₀ alkyl, C₂-C₁₀ alkenyl, C₂-C₁₀ alkynyl, -(CH₂)ₜ(C₆-C₁₀ aryl), or -(CH₂)ₜ(5-10 membered heterocyclic), wherein t is an integer from 0 to 5; said alkyl group optionally includes 1 or 2 hetero moieties selected from O, S and -N(R⁶)- with the proviso that two O atoms, two S atoms, or an O and S atom are not attached directly to each other; said aryl and heterocyclic R³ groups are optionally fused to a C₆-C₁₀ aryl group, a C₅-C₈ saturated cyclic group, or a 5-10 membered heterocyclic group; 1 or 2 carbon atoms in the foregoing heterocyclic moieties are optionally substituted by an oxo (=O) moiety; the -(CH₂)ₜ- moieties of the foregoing R³ groups optionally include a carbon-carbon double or triple bond where t is an integer from 2 to 5, and the foregoing R³ groups are optionally substituted by 1 to 5 R⁴ groups;
each R⁴ is independently selected from C₁-C₁₀ alkyl, C₂-C₁₀ alkenyl, C₂-C₁₀ alkynyl, halo, cyano, nitro, trifluoromethyl, trifluoromethoxy, azido, -OR⁵, -C(O)R⁵, -C(O)OR⁵, -NR⁶C(O)OR⁵, -OC(O)R⁵, -NR⁶SO₂R⁵, -SO₂NR⁵R⁶, -NR⁶C(O)R⁵, -C(O)NR⁵R⁶, -NR⁵R⁶, -S(O)ⱼR⁷ wherein j is an integer ranging from 0 to 2, -SO₃H, -NR⁵(CR⁶R⁷)ₜOR⁶, -(CH₂)ₜ(C₆-C₁₀ aryl), -SO₂(CH₂)ₜC₆-C₁₀ aryl), -S(CH₂)ₜC₆-C₁₀ aryl), -O(CH₂)ₜ(C₆-C₁₀ aryl), -(CH₂)ₜ(5-10 membered heterocyclic), and -(CR⁶R⁷)ₘOR⁶, wherein m is an integer from 1 to 5 and t is an integer from 0 to 5; said alkyl group optionally contains 1 or 2 hetero moieties selected from O, S and -N(R⁶)-with the proviso that two O atoms, two S atoms, or an O and S atom are not attached directly to each other; said aryl and heterocyclic R⁴ groups are optionally fused to a C₆-C₁₀ aryl group, a C₅-C₈ saturated cyclic group, or a 5-10 membered heterocyclic group; 1 or 2 carbon atoms in the foregoing heterocyclic moieties are optionally substituted by an oxo (=O) moiety; and the alkyl, aryl and heterocyclic moieties of the foregoing R⁴ groups are optionally substituted by 1 to 3 substituents independently selected from halo, cyano, nitro, trifluoromethyl, trifluoromethoxy, azido, -NR⁶SO₂R⁵, -SO₂NR⁵R⁶, -C(O)R⁵, -C(O)OR⁵, -OC(O)R⁵, -NR⁶C(O)R⁵, -C(O)NR⁵R⁶, -NR⁵R⁶, -(CR⁶R⁷)ₘOR⁶ wherein m is an integer from 1 to 5, -OR⁵ and the substituents listed in the definition of R⁵;

Each R⁵ is independently selected from H, C₁-C₁₀ alkyl, -(CH₂)ₜ(C₆-C₁₀ aryl), and -(CH₂)ₜ(5-1 membered heterocyclic), wherein t is an integer from 0 to 5; said alkyl group optionally includes 1 or 2 hetero moieties selected from O, S and -N(R⁶)- with the proviso that two O atoms, two S atoms, or an O and S atom are not attached directly to each other; said aryl and heterocyclic R⁵ groups are optionally fused to a C₆-C₁₀ aryl group, a C₅-C₈ saturated cyclic group, or a 5-10 membered heterocyclic group; and the foregoing R⁵ substituents, except H, are optionally substituted by 1 to 3 substituents independently selected from halo, cyano, nitro, trifluoromethyl, trifluoromethoxy, azido, -C(O)R⁶, -C(O)OR⁶, -CO(O)R⁶, -NR⁶C(O)R⁷, - C(O)NR⁶R⁷, -NR⁶R⁷, hydroxy, C₁-C₆ alkyl, and C₁-C₆ alkoxy; and,
each R⁶ and R⁷ is independently H or C₁-C₆ alkyl;
for the manufacture of pharmaceutials for simultaneous or sequential administration for treating hyperproliferative disorders.

In one embodiment of the use of the present invention the hyperproliferative disorder is cancer, wherein said cancer is selected from the group consisting of brain, squamous cell, bladder, gastric, pancreatic, breast, head, neck, oesophageal, prostate, colorectal, lung, renal, kidney, ovarian, gynecological and thyroid cancer.

In one preferred embodiment the cancer is selected from the group consisting of prostate, breast, lung, colon and ovarian cancer.

In a more preferred embodiment the cancer is selected from the group consisting of prostate, breast, and lung cancer.

In one preferred embodiment the breast cancer is metastatic breast cancer.

In another preferred embodiment the lung cancer is non-small cell lung cancer (NSCL).

In another embodiment of the use of the present invention the hyperproliferative disorder is non-cancerous.

In one embodiment the non-cancerous hyperproliferative disorder is benign hyperplasia of the skin or prostate.

Preferred compounds include those of formula 1 wherein R² is H and R¹ is C₁-C₁₀ alkyl optionally substituted by 1 or 2 substituents independently selected from -NR⁵R⁶, -NR⁵(CR⁶R⁷)ₜOR⁶ and -(CH₂)ₜ(5-10 membered heterocyclic) wherein t is an integer from 0 to 5. Specific preferred R¹ groups include propyl, butyl, pentyl and hexyl optionally substituted by dimethylamino, hydroxy, pyrrolidinyl, morpholino, and ethyl-(2-hydroxy-ethyl)-amino.

Other preferred compounds include those of formula 1 wherein R² is H and R¹ is -(CH₂)ₜ(5-10 membered heterocyclic), wherein t is an integer from 0 to 5; said heterocyclic group is optionally fused to a C₆-C₁₀ aryl group, a C₅-C₈ saturated cyclic group, or a 5-10 membered heterocyclic group; and said R¹ group, including the optionally fused portions of said R¹ group, is optionally substituted by 1 or 2 substituents independently selected from C₁-C₄ alkyl, hydroxy and hydroxymethyl. Specific preferred heterocyclic groups of said R¹ group are morpholino, pyrrolidinyl, imidazolyl, piperazinyl, piperidinyl, and 2,5-diaza-bicyclo[2.2.1]hept-2-yl, the t variable of said R¹ group ranges from 2 to 5, and said heterocyclic groups are optionally substituted by hydroxy, hydroxymethyl and methyl.

Other preferred compounds include those of formula 1 wherein R³ is -(CH₂⁾t(C₆-C₁₀ aryl) wherein t is an integer from 1 to 3 and said R³ group is optionally substituted by 1 to 4 R⁴ groups. Specific preferred R³ groups include benzyl optionally substituted by 1 to 4 substituents independently selected from halo and C₁-C₄ alkyl. More specific preferred R³ groups include benzyl substituted by 1 to 4 substituents independently selected from methyl, fluoro, chloro and bromo.

Specific embodiments of the present invention include the following compounds:
5-{3-[3-(4-Methyl-piperazin-1-yl)-propyl]-ureido}-3-(2,3,6-trifluoro-4-methyl-benzyloxy)-isothiazole-4-carboxylic acid amide;
3-(4-Chloro-2,6-difluoro-benzyloxy)-5-(3-{4-[ethyl-(2-hydroxy-ethyl)-amino]-butyl}-ureido)-isothiazole-4-carboxylic acid amide;
3-(2-Fluoro-4-methyl-benzyloxy)-5-{3-[3-(4-methyl-piperazin-1-yl)-propyl]-ureido}-isothiazole-4-carboxylic acid amide;
3-(2,5-Difluoro-4-methyl-benzyloxy)-5-(3-4-[4-(2-hydroxy-ethyl)-piperazin-1-yl]-butyl}-ureido)-isothiazole-4-carboxylic acid amide;
3-(2,5-Difluoro-4-methyl-benzyloxy)-5-[3-(6-dimethylamino-hexyl)-ureido]-isothiazole-4-carboxylic acid amide;
3-(2-Fluoro-4-methyl-benzyloxy)-5-[3-(5-isopropylamino-pentyl)-ureido]-isothiazole-4-carboxylic acid amide;
3-(4-Chloro-2,6-difluoro-benzyloxy)-5-[3-(4-pyrrolidin-1-yl-butyl)-ureido]-isothiazole-4-carboxylic acid amide;
3-(4-Chloro-2,6-difluoro-benzyloxy)-5-{3-[3-(4-methyl-piperazin-1-yl)-propyl]-ureido}-isothiazole-4-carboxylic acid amide;
3-(4-Chloro-2,6-difluoro-benzyloxy)-5-{3-[-(1-methyl-pyrrolidin-2-yl)-ethyl]-ureido}-isothiazole-4-carboxylic acid amide;
3-(2,6-Difluoro-4-methyl-benzyloxy)-5-[3-(4-pyrrolidin-1-yl-butyl)-ureido]-isothiazole-4-carboxylic acid amide;
3-(2,6-Difluoro-4-methyl-benzyloxy)-5-[3-{4-[4-(2-hydroxy-ethyl)-piperazin-1-yl]-butyl}-ureido)-isothiazole-4-carboxylic acid amide;
3-(4-Chloro-2,6-difluoro-benzyloxy)-5-[3-(3-hydroxy-5-pyrrolidin-1-yl)-pentyl)-ureido}-isothiazole-4-carboxylic acid amide;
3-(2,5-Difluoro-4-methyl-benzyloxy)-5-{3-[4-(3,4-dihydroxy-pyrrolidin-1-yl]-butyl]-ureido}-isothiazole-4-carboxylic acid amide;
3-(4-Chloro-2,6-difluoro-benzyloxy)-5-{3-[4-(3,4-dihydroxy-pyrrolidin-1-yl)-butyl]-ureido}- isothiazole-4-carboxylic acid amide;
3-(2,5-Difluoro-4-methyl-benzyloxy)-5-{3-[4-(2-hydroxymethyl-pyrrolidin-1-yl)-butyl]-ureido}-isothiazole-4-carboxylic acid amide;
3-(4-Chloro-2,6-difluoro-benzyloxy)-5-{3-[4-(2-hydroxymethyl-pyrrolidin-1-yl)-butyl]-ureido}-isothiazole-4-carboxylic acid amide;
3-(2,5-Difluoro-4-methyl-benzyloxy)-5-{3-[4-(3-hydroxy-pyrrolidin-1-yl)-butyl]-ureido}-isothiazole-4-carboxylic acid amide;
3-(4-Bromo-2,6-difluoro-benzyloxy)-5-[3-(4-pyrrolidin-1-yl-butyl)-ureido}-isothiazole-4-carboxylic acid amide;
mesylate salt of 3-(4-Bromo-2,6-difluoro-benzyloxy)-5-[3-(4-pyrrolidin-1-yl-butyl)-ureido}-isothiazole-4-carboxylic acid amide;
3-(2,6-Difluoro-4-methyl-benzyloxy)-5-[3-(4-hydroxy-5-piperidin-1-yl-pentyl)-ureido]-isothiazole-4-carboxylic acid amide;
3-(2,5-Difluoro-4-methyl-benzyloxy)-5-{3-[4-(3-hydroxy-5-piperidin-1-yl-pentyl)-ureido]-isothiazole-4-carboxylic acid amide;
3-(4-Chloro-2,6-difluoro-benzyloxyr5-{3-[4-(2-hydroxymethyl-piperidin-1-yl)-butyl]-ureido}-isothiazole-4-carboxylic acid amide;
3-(2,5-Difluoro-4-methyl-benzyloxy)-5-(3-{4-[ethyl-(2-hydroxy-ethyl)-amino]-butyl}-ureido)-isothiazole-4-carboxylic acid amide;
3-(4-Chloro-2,6-difluoro-benzyloxy)-5-[3-(5-hydroxy-6-piperidin-1-yl)-hexyl)-ureido}-isothiazole-4-carboxylic acid amide;
3-(4-Bromo-2,3,6-trifluoro-benzyloxy)-5-{3-[3-(4-methyl-piperazin-1-yl)-propyl]-ureido}-isothiazole-4-carboxylic acid amide;
3-(2,6-Difluoro-4-methyl-benzyloxy)-5-{3-[3-(4-methyl-piperazin-1-yl-propyl]-ureido}-isothiazole-4-carboxylic acid amide;
hydrochloride salt of 3-(4-Bromo-2,6-difluoro-benzyloxy)-5-[3-(4-pyrrolidin-1-yl-butyl)-ureido}-isothiazole-4-carboxylic acid amide;
3-(2,6-Difluoro-4-methyl-benzyloxy)-5-[3-(3-hydroxy-5-pyrrolidin-1-yl-pentyl)-ureido]-isothiazole-4-carboxylic acid amide;
5-[3-(4-Pyrrolidin-1-yl-butyl)-ureido]-3-(2,3,6-trifluoro-4-methyl-benzyloxy)-isothiazole-4-carboxylic acid amide;
5-[3-(3-Hydroxy-5-pyrrolidin-1-yl-pentyl)-ureido]-3-(2,3,6-trifluoro-4-methyl-benzyloxy)-isothiazole-4-carboxylic acid amide;
3-(4-Chloro-2,6-difluoro-benzyloxy)-5-{3-[3-(5-methyl-2,5-diazabicyclo[2.2.1]hept-2-yl)-propyl]-ureidol-isothiazole-4-carboxylic acid amide;
3-(4-Chloro-2,3,6-trifluoro-benzyloxy)-5-{3-[3-(5-methyl-2,5-diaza-bicyclo[2.2.1]hept-2-yl)-propyl]-ureido}-isothiazole-4-carboxylic acid amide;
3-(4-Chloro-2,3,6-trifluoro-benzyloxy)-5-{3-[2-(1-methyl-pyrrolidin-2-yl)-ethyl]-ureido}-isothiazole-4-carboxylic acid amide;
3-(4-Chloro-2,3,6-trifluoro-benzyloxy)-5-[3-(4-pyrrolidin-1-yl-butyl)-ureido]-isothiazole-4-carboxylic acid amide;
3-(4-Chloro-2,3,6-trifluoro-benzyloxy)-5-{3-[4-(2-hydroxymethyl-pyrrolidin-1-yl)-butyl]-ureido}-isothiazole-4-carboxylic acid amide;
5-{3-[2-(1-Methyl-pyrrolidin-2-yl)-ethyl]-ureido}-3-(2,3,6-trifluoro-4-methyl-benzyloxy)-isothiazole-4-carboxylic acid amide;
5-[3-(4-Dimethylamino-butyl)-ureido]-3-(2,3,6-trifluoro-4-methyl-benzyloxy)-isothiazole-4-carboxylic acid amide;
5-[3-(3-Dimethylamino-propyl)-ureido]-3-(2,3,6-trifluoro-4-methyl-benzyloxy)-isothiazole-4-carboxylic acid amide;
5-[3-(3-Hydroxy-5-isopropropylam ino-pentyl)-ureido]-3-(2,3,6-trifluoro-4-methyl-benzyloxy)-isothiazole-4-carboxylic acid amide;
5-[3-(3-Isopropylamino-propyl)-ureido]-3-(2,3,6-trifluoro-4-methyl-benzyloxy)-isothiazole-4-carboxylic acid amide;
5-{3-[4-(4-Methyl-piperazin-1-yl)-butyl]-ureido}-3-(2,3,6-trifluoro-4-methyl-benzyloxy)-isothiazole-4-carboxylic acid amide;
5-(3-{4-[4-(2-Hydroxy-ethyl)-piperazin-1-yl]-butyl}-ureido)-3-(2,3,6-trifluoro-4-methyl-benzyloxy)-isothiazole-4-carboxylic acid amide;
5-[3-(3-Pyrrolidin-1-yl-propyl)-ureido]-3-(2,3,6-trifluoro-4-methyt-benzyloxy)-isothiazole-4-carboxylic acid amide;
5-[3-(4-Hydroxy-5-piperidin-1-yl-pentyl)-ureido]-3-(2,3,6-trifluoro-4-methyl-benzyloxy)-isothiazole-4-carboxylic acid amide;
3-(4Chloro-2,6-difluoro-benzyloxy)-5-[3-(4-imidazol-1-yl-butyl)-ureido]-isothiazole-4-carboxylic acid amide;
5-(3-{4-[Ethyl-(2-hydroxy-ethyl)-amino]-butyl}-ureido)-3-(2,3,6-trifluoro-4-methyl-benzyloxy)-isothiazole-4-carboxylic acid amide;
3-(4-Chloro-(2,3,6-trifluoro-benzyloxy)-5-{3-[4-(2-hydroxmethyl-piperidin-1-yl)-butyl]-ureido}-isothiazole-4-carboxylic acid amide;
3-(4-Chloro-2,3,6-trifluoro-benzyloxy)-5-[3-(3-hydroxy-5-pyrrolidin-1-yl-pentyl)-ureido]-isothiazole-4-carboxylic acid amide;
3-(4-Bromo-2,6-difluoro-benzyloxy)-5-{3-[3-(4-methyl-piperazin-1-yl)-propyl]-ureido}-isothiazole-4-carboxylic acid amide;
3-(2,6-Difluoro-4-methyl-benzyloxy)-5-{3-[2-(1-methyl-pyrrolidin-2-yl)-ethyl]-ureido}-isothiazole-4-carboxylic acid amide;
3-(2,6-Difluoro-4-methyl-benzyloxy)-5-[3-(4-dimethylamino-butyl)-ureido}-isothiazole-4-carboxylic acid amide;
3-(2,6-Difluoro-4-methyl-benzyloxy)-5-[3-(3-dimethylamino-propyl)-ureido]-isothiazole-4-carboxylic acid amide;
3-(4-Bromo-2,3,6-trifluoro-benzyloxy)-5-[3-(4-pyrrolidin-1-yl-butyl)-ureido]-isothiazole-4-carboxylic acid amide;
3-(4-Chloro-2,3,6-trifluoro-benzyloxy)-5-[3-(4-imidazol-1-yl-butyl)-ureido]-isothiazole-4-carboxylic acid amide;
3-(4-Chloro-2,3,6-difluoro-benzyloxy)-5-(3-{3-[ethyl-(2-hydroxy-ethyl)-amino]-propyl}-ureido)-isothiazole-4-carboxylic acid amide;
3-(4-Chloro-2,3,6-trifluoro-benzyloxy)-5-(3-{3-[ethyl-(2-hydroxy-ethyl)-amino]-propyl}-ureido)-isothiazole-4-carboxylic acid amide;
5-[3-(3-Methylamino-propyl)-ureido]-3-(2,3,6-trifluoro-4-methyl-benzyloxy)-isothiazole-4-carboxylic acid amide;
5-[3-(3-Amino-propyl)-3-m ethyl-ureido]-3-(2,3,6-trifluoro-4-methyl-benzyloxy)-isothiazole-4-carboxylic acid amide;
5-[3-(4-Diethylamino-butyl)-ureido]-3-(2,3,6-trifluoro-4-methyl-benzyloxy)-isothiazole-4-carboxylic acid amide;
3-(2,6-Difluoro-4-methyl-benzyloxy)-5-[3-(3-pyrrolidin-1-yl-propyl)-ureido]-isothiazole-4-carboxylic acid amide;
3-(3-Chloro-2,6-difluoro-4-methyl-benzyloxy)-5-[3-(4-dimethylamino-butyl)-ureido]-isothiazole-4-carboxylic acid amide;
5-(3-{4-[Bis-(2-hydroxy-ethyl)-amino]-butyl}-ureido)-3-(2,6-difluoro-4-methyl-benzyloxy)-isothiazole-4-carboxylic acid amide;
and the pharmaceutically acceptable salts and hydrates of the foregoing compounds.

Preferred embodiments of the present invention include the following compounds:
3-(4-Bromo-2,6-difluoro-benzyloxy)-5-[3-(4-pyrrolidin-1-yl-butyl)-ureido]-isothiazole-4-carboxylic acid amide
mesylate salt of 3-(4-Bromo-2,6-difluoro-benzyloxy)-5-[3-(4-pyrrolidin-1-yl-butyl)-ureidol-isothiazole-4-carboxylic acid amide;
5-{3-[3-(4-Methyl-piperazin-1-yl)-propyl]-ureido}-3-(2,3,6-trifluoro-4-methyl-benzyloxy)-isothiazole-4-carboxylic acid amide;
3-(4-Chloro-2,6-difluoro-benzyloxy)-5-(3-{4-[ethyl-(2-hydroxy-ethyl)-amino]-butyl}-ureido)-isothiazole-4-carboxylic acid amide;
3-(2-Fluoro-4-methyl-benzyloxy)-5-{3-[3-(4-methyl-piperazin-1-yl)-propyl]-ureido}-isothiazole-4-carboxylic acid amide;
3-(2,5-Difluoro-4-methyl-benzyloxy)-5-(3-4-[4-(2-hydroxy-ethyl)-piperazin-1-yl]-butyl}-ureido)-isothiazole-4-carboxylic acid amide;
3-(2,5-Difluoro-4-methyl-benzyloxy)-5-[3-(6-dimethylamino-hexyl)-ureido]-isothiazole-4-carboxylic acid amide;
3-(2-Fluoro-4-methyl-benzyloxy)-5-[3-(5-isopropylamino-pentyl)-ureido]-isothiazole-4-carboxylic acid amide;
hydrochloride salt of 3-(4-Bromo-2,6-difluoro-benzyloxy)-5-[3-(4-pyrrolidin-1-yl-butyl)-ureido}-isothiazole-4-carboxylic acid amide;
and the pharmaceutically acceptable salts, prodrugs and solvates of said compounds.

More preferred embodiments of the present invention include compounds of formula 1 is selected from the group consisting of
3-(4-Bromo-2,6-difluoro-benzyloxy)-5-[3-(4-pyrrolidin-1-yl-butyl)-ureido]-isothiazole-4-carboxylic acid amide
mesylate salt of 3-(4-Bromo-2,6-difluoro-benzyloxyr5-[3-(4-pyrrolidin-.1-yl-butyl)-ureido}-isothiazole-4-carboxylic acid amide;
hydrochloride salt of 3-(4-Bromo-2,6-difluoro-benzyloxy)-5-[3-(4-pyrrolidin-1-yl-butyl)-ureido}-isothiazole-4-carboxylic acid amide;
and the pharmaceutically acceptable salts and solvates of said compounds.

In a more preferred embodiment of the present invention the compound of formula 1 is a hydrochloride salt of 3-(4-Bromo-2,6-difluoro-benzyloxy)-5-[3-(4-pyrrolidin-1-yl-butyl)-ureido}-isothiazole-4-carboxylic acid amide; and the pharmaceutically acceptable salts and solvates of said compound.

The present invention also relates to a pharmaceutical composition for the treatment of a hyperproliferative disorder in a mammal which comprises (i) gemcitabine hydrochloride; and (ii) a therapeutically effective amount of a compound of the formula 1, in combination with one or more pharmaceutically acceptable carriers or vehicles.

In one embodiment, said pharmaceutical composition is for the treatment of cancer such as brain, lung, squamous cell, bladder, gastric, pancreatic, breast, head, neck, renal, prostate, colorectal, oesophageal, gynecological (such as ovarian) or thyroid cancer. In another embodiment, said pharmaceutical composition is for the treatment of a non-cancerous hyperproliferative disorder such as benign hyperplasia of the skin (e.g., psoriasis) or prostate (e.g., benign prostatic hypertrophy (BPH)).

In one preferred embodiment the pharmaceutical composition is for the treatment of cancer selected from brain, squamous cell, bladder, gastric, pancreatic, breast, head, neck, oesophageal, prostate, colorectal, lung, renal, kidney, ovarian, gynecological and thyroid cancer. In a more preferred embodiment the pharmaceutical composition is for the treatment of prostate, breast, lung, colon and ovarian cancer. In an even more preferred embodiment the pharmaceutical composition is for the treatment of prostate, breast, and lung cancer. In a most preferred embodiment the pharmaceutical composition is for the treatment of metastatic breast cancer or NSCL.

The invention also relates to a pharmaceutical composition as set out above for the treatment of pancreatitis or kidney disease (including proliferative glomerulonephritis and diabetes-induced renal disease) in a mammal; or
for the prevention of blastocyte implantation in a mammal; or
for treating a disease related to vasculogenesis or angiogenesis in a mammal.

In one embodiment, said pharmaceutical composition is for treating a disease selected from the group consisting of tumor angiogenesis, chronic inflammatory disease such as rheumatoid arthritis, atherosclerosis, skin diseases such as psoriasis, eczema, and scleroderma, diabetes, diabetic retinopathy, retinopathy of prematurity, age-related macular degeneration, hemangioma, glioma, melanoma, Kaposi's sarcoma and ovarian, breast, lung, pancreatic, prostate, colon and epidermoid cancer.

In one embodiment, the use of the present invention relates to the treatment of cancer such as brain, squamous cell, bladder, gastric, pancreatic, breast, head, neck, oesophageal, prostate, colorectal, lung, renal, gynecological (such as ovarian) or thyroid cancer. In another embodiment, said method relates to the treatment of a non-cancerous hyperproliferative disorder such as benign hyperplasia of the skin (e.g., psoriasis) or prostate (e.g., benign prostatic hypertrophy (BPH)).

The invention also relates to the use of (i) a therapeutically effective amount of gemcitabine hydrochloride and (ii) a therapeutically effective amound of a compound of the formula 1 or a Pharmaceutically acceptable salt or solvate thereof in combination with one or more pharmaceutically acceptable carriers or vehicles in the manufacture of a pharmaceutical for simultaneous or sequential administration for treating pancreatitis or kidney disease in a mammal; preventing blastocyte implantation in a mammal; treating diseases related to vasculogenesis or angiogenesis in a mammal;

In one embodiment, the disease is selected from the group consisting of tumor angiogenesis, chronic inflammatory disease such as rheumatoid arthritis, atherosclerosis, skin diseases such as psoriasis, eczema, and scleroderma, diabetes, diabetic retinopathy, retinopathy of prematurity, macular degeneration, hemangioma, glioma, melanoma, Kaposi's sarcoma and ovarian, breast, lung, pancreatic, prostate, colon and epidermoid cancer.

Patients that can be treated with compounds of formulas 1 and the pharmaceutically acceptable salts and hydrates of said compounds, in combination with gemcitabine hydrochloride according to the use of this invention include, for example, patients that have been diagnosed as having psoriasis, BPH, lung cancer, bone cancer, pancreatic cancer, skin cancer, cancer of the head and neck, cutaneous or intraocular melanoma, uterine cancer, ovarian cancer, rectal cancer or cancer of the anal region, stomach cancer, colon cancer, breast cancer, gynecologic tumors (e.g., uterine sarcomas, carcinoma of the fallopian tubes, carcinoma of the endometrium, carcinoma of the cervix, carcinoma of the vagina or carcinoma of the vulva), Hodgkin's disease, cancer of the esophagus, cancer of the small intestine, cancer of the endocrine system (e.g., cancer of the thyroid, parathyroid or adrenal glands), sarcomas of soft tissues, cancer of the urethra, cancer of the penis, prostate cancer, chronic or acute leukemia, solid tumors of childhood, lymphocytic lymphomas, cancer of the bladder, cancer of the kidney or ureter (e.g., renal cell carcinoma, carcinoma of the renal pelvis), or neoplasms of the central nervous system (e.g., primary CNS lymphoma, spinal axis tumors, brain stem gliomas or pituitary adenomas).

The present invention also relates to a kit comprising in a first compartment a compound of formula 1 and in a second compartment gemcitabine hydrochloride.

The terms "concurrently" and "simultaneously" are used interchangeably and mean the compounds of the combination therapy of the present invention are administered (1) simultaneously in time, or (2) at different times during the course of a common treatment schedule.

The term "sequentially" as used herein means (1) administration of one component of the method ((i) a compound of formula 1 or (ii) gemcitabine hydrochloride) followed by administration of the other component; after administration of one component, the second component can be administered substantially immediately after the first component, or the second component can be administered after an effective time period after the first component; the effective time period is the amount of time given for realization of maximum benefit from the administration of the first component.

The term "halo", as used herein, unless otherwise indicated, includes fluoro, chloro, bromo or iodo. Preferred halo groups are fluoro, chloro and bromo.

The term "alkyl", as used herein, unless otherwise indicated, includes saturated monovalent hydrocarbon radicals having straight, cyclic or branched moieties. It is understood that for cyclic moieties at least three carbon atoms are required in said alkyl group.

The term "alkenyl", as used herein, unless otherwise indicated, includes monovalent hydrocarbon radicals having at least one carbon-carbon double bond and also having straight, cyclic or branched moieties as provided above in the definition of "alkyl".

The term "alkynyl", as used herein, unless otherwise indicated, includes monovalent hydrocarbon radicals having at least one carbon-carbon triple bond and also having straight, cyclic or branched moieties as provided above in the definition of "alkyl".

The term "alkoxy", as used herein, unless otherwise indicated, includes O-alkyl groups wherein "alkyl" is as defined above.

The term "aryl", as used herein, unless otherwise indicated, includes an organic radical derived from an aromatic hydrocarbon by removal of one hydrogen, such as phenyl or naphthyl.

The term "4-10 membered heterocyclic", as used herein, unless otherwise indicated, includes aromatic and non-aromatic heterocyclic groups containing one or more heteroatoms each selected from O, S and N, wherein each heterocyclic group has from 4-10 atoms in its ring system. Non-aromatic heterocyclic groups include groups having only 4 atoms in their ring system, but aromatic heterocyclic groups must have at least 5 atoms in their ring system. An example of a 4 membered heterocyclic group is azetidinyl (derived from azetidine). An example of a 5 membered heterocyclic group is thiazolyl and an example of a 10 membered heterocyclic group is quinolinyl. Examples of non-aromatic heterocyclic groups are pyrrolidinyl, tetrahydrofuranyl, tetrahydrothienyl, tetrahydropyranyl, tetrahydrothiopyranyl, piperidino, morpholino, thiomorpholino, thioxanyl, piperazinyl, azetidinyl, oxetanyl, thietanyl, homopiperidinyl, oxepanyl, thiepanyl, oxazepinyl, diazepinyl, thiazepinyl, 1,2,3,6-tetrahydropyridinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinyl, imidazolinyl, imidazolidinyl, 3-azabicyclo[3.1.0]hexanyl, 3-azabicyclo[4.1.0]heptanyl, 3H-indolyl and quinolizinyl. Examples of aromatic heterocyclic groups are pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, thiadiazolyl, furazanyl, benzofurazanyl, benzothiophenyl, benzothiazolyl, benzoxazolyl, quinazolinyl, quinoxalinyl, naphthyridinyl, and furopyridinyl. The foregoing groups, as derived from the compounds listed above, may be C-attached or N-attached where such is possible. For instance, a group derived from pyrrole may be pyrrol-1-yl (N-attached) or pyrrol-3-yl (C-attached).

The phrase "pharmaceutically acceptable salt(s)", as used herein, unless otherwise indicated, includes salts of acidic or basic groups which may be present in the compounds of formula 1. The compounds of formula 1 that are basic in nature are capable of forming a wide variety of salts with various inorganic and organic acids. The acids that may be used to prepare pharmaceutically acceptable acid addition salts of such basic compounds of formula 1 are those that form non-toxic acid addition salts, i.e., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate and pamoate i.e., 1,1'-methylene-bis-(2-hydroxy-3-naphthoate)] salts.

Those compounds of the formula 1 that are acidic in nature, are capable of forming base salts with various pharmacologically acceptable cations. Examples of such salts include the alkali metal or alkaline earth metal salts and particularly, the sodium and potassium salts.

Certain compounds of formula 1 may have asymmetric centers and therefore exist in different enantiomeric forms. This invention relates to the use of all optical isomers and stereoisomers of the compounds of formula 1 and mixtures thereof. The compounds of formula 1 may also exist as tautomers. This invention relates to the use of all such tautomers and mixtures thereof.

The subject invention also includes isotopically-labelled compounds, and the pharmaceutically acceptable salts thereof, which are identical to those recited in formula 1, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine and chlorine, such as ²H , ³H, ¹³C, ¹⁴C, ¹⁵N, ¹⁸O, ¹⁷O, ³⁵S, ¹⁸F, and ³⁸Cl, respectively. Compounds of the present invention, prodrugs thereof, and pharmaceutically acceptable salts of said compounds or of said prodrugs which contain the aforementioned isotopes and/or other isotopes of other atoms are within the scope of this invention. Certain isotopically-labelled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labelled compounds of formula 1 of this invention and prodrugs thereof can generally be prepared by carrying out the procedures disclosed in the Schemes and/or in the Examples and Preparations below, by substituting a readily available isotopically labelled reagent for a non-isotopically labelled reagent.

Compounds of formula 1 having free amino, amido, hydroxy or carboxylic groups can be converted into prodrugs. Prodrugs include compounds wherein an amino acid residue, or a polypeptide chain of two or more (e.g., two, three or four) amino acid residues is covalently joined through an amide or ester bond to a free amino, hydroxy or carboxylic acid group of compounds of formula 1. The amino acid residues include the 20 naturally occurring amino acids commonly designated by three letter symbols and also includes 4-hydroxyproline, hydroxylysine, demosine, isodemosine, 3-methylhistidine, norvalin, beta-alanine, gamma-aminobutyric acid, citrulline homocysteine, homoserine, ornithine and methionine sulfone.

Additional types of prodrugs are also encompassed. For instance, free carboxyl groups can be derivatized as amides or alkyl esters. The amide and ester moieties may incorporate groups including but not limited to ether, amine and carboxylic acid functionalities. Free hydroxy groups may be derivatized using groups including hemisuccinates, phosphate esters, dimethylaminoacetates, and phosphoryloxymethyloxycarbonyls, as outlined in D. Fleisher, R. Bong, B.H. Stewart, Advanced Drug Delivery Reviews (1996) 19, 115. Carbamate prodrugs of hydroxy and amino groups are also included, as are carbonate prodrugs and sulfate esters of hydroxy groups. Derivatization of hydroxy groups as (acyloxy)methyl and (acyloxy)ethyl ethers wherein the acyl group may be an alkyl ester, optionally substituted with groups including ether, amine and carboxylic acid functionalities, or where the acyl group is an amino acid ester as described above, are also encompassed. Prodrugs of this type are described in R.P. Robinson et al., J. Medicinal Chemistry (1996) 39, 10.

### Detailed Description of the Invention

The present invention relates to the use of (i) a therapeutically effective amount of gemcitabine hydrochloride; and (ii) a therapeutically effective amount of a compound of the formula 1 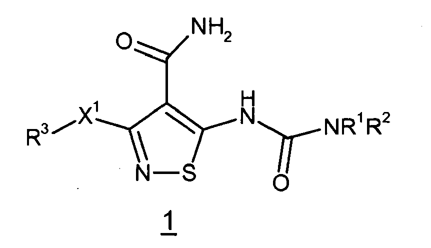 or a pharmaceutically acceptable salt or solvate thereof, wherein:
wherein X¹ is O or S;
R¹ is H, C₁-C₁₀ alkyl, C₂-C₁₀ alkenyl, C₂-C₁₀ alkynyl, -C(O)(C₁-C₁₀ alkyl), -(CH₂)ₜ(C₆-C₁₀ aryl), -(CH₂)ₜ(4-10 membered heterocyclic), -C(O)(CH₂)ₜC₆-C_{1Q} aryl), or -C(O)(CH₂)ₜ(5-10 membered heterocyclic), wherein t is an integer from 0 to 5; said alkyl group optionally includes 1 or 2 hetero moieties selected from O, S and -N(R⁶)- with the proviso that two O atoms, two S atoms, or an O and S atom are not attached directly to each other; said aryl and heterocyclic R¹ groups are optionally fused to a C₆-C₁₀ aryl group, a C₅-C₈ saturated cyclic group, or a 5-10 membered heterocyclic group; 1 or 2 carbon atoms in the foregoing heterocyclic moieties are optionally substituted by an oxo (=O) moiety; the -(CH₂)ₜ- moieties of the foregoing R¹ groups optionally include a carbon-carbon double or triple bond where t is an integer from 2 to 5; and the foregoing R¹ groups, except H, are optionally substituted by 1 to 3 R⁴ groups;
R² is selected from the list of substituents provided in the definition of R¹, -SO₂(CH₂)ₜ(C₆-C₁₀ aryl), -SO₂(CH₂)ₜ(5-10 membered heterocyclic), and -OR⁵, t is an integer ranging from 0 to 5, the -(CH₂)ₜ- moieties of the foregoing R² groups optionally include a carbon-carbon double or triple bond where t is an integer from 2 to 5, and the foregoing R² groups are optionally substituted by 1 to 3 R⁴ groups;
or R¹ and R² may be taken together with the nitrogen to which each is attached to form a 4-10 membered saturated monocyclic or polycyclic ring or a 5-10 membered heteroaryl ring, wherein said saturated and heteroaryl rings optionally include 1 or 2 heteroatoms selected from O, S and -N(R⁶)- in addition to the nitrogen to which R¹ and R² are attached,
said -N(R⁶)- is optionally =N- or -N= where R¹ and R² are taken together as said heteroaryl group, said saturated ring optionally may be partially unsaturated by including 1 or 2 carbon-carbon double bonds, and said saturated and heteroaryl rings, including the R⁶ group of said -N(R⁶)-, are optionally substituted by 1 to 3 R⁴ groups;
R³ is H, C₁-C₁₀ alkyl, C₂-C₁₀ alkenyl, C₂-C₁₀ alkynyl, -(CH₂)ₜ(C₆-C₁₀ aryl), or -(CH₂)ₜ(5-10 membered heterocyclic), wherein t is an integer from 0 to 5; said alkyl group optionally includes 1 or 2 hetero moieties selected from O, S and -N(R⁶)- with the proviso that two O atoms, two S atoms, or an O and S atom are not attached directly to each other; said aryl and heterocyclic R³ groups are optionally fused to a C₆-C₁₀ aryl group, a C₅-C₈ saturated cyclic group, or a 5-10 membered heterocyclic group; 1 or 2 carbon atoms in the foregoing heterocyclic moieties are optionally substituted by an oxo (=O) moiety; the -(CH₂)ₜ- moieties of the foregoing R³ groups optionally include a carbon-carbon double or triple bond where t is an integer from 2 to 5, and the foregoing R³ groups are optionally substituted by 1 to 5 R⁴ groups;
each R⁴ is independently selected from C₁-C₁₀ alkyl, C₂-C₁₀ alkenyl, C₂-C₁₀ alkynyl, halo, cyano, nitro, trifluoromethyl, trifluoromethoxy, azido, -OR⁵, -C(O)R⁵, -C(O)OR⁵, -NR⁶C(O)OR⁵, -OC(O)R⁵, -NR⁶SO₂R⁵, -SO₂NR⁵R⁶, -NR⁶C(O)R⁵, -C(O)NR⁵R⁶, -NR⁵R⁶, -S(O)ⱼR⁷ wherein j is an integer ranging from 0 to 2, -SO₃H, -NR⁵(CR⁶R⁷)ₜOR⁶, -(CH₂)ₜ(C₆-C₁₀ aryl), -SO₂(CH₂)ₜ(C₆-C₁₀ aryl), -S(CH₂)ₜ(C₆-C₁₀ aryl), -O(CH₂)ₜ(C₆-C₁₀ aryl), -(CH₂⁾t(5-10 membered heterocyclic), and -(CR⁶R⁷)ₘOR⁶, wherein m is an integer from 1 to 5 and t is an integer from 0 to 5; said alkyl group optionally contains 1 or 2 hetero moieties selected from O, S and -N(R⁶)-with the proviso that two O atoms, two S atoms, or an O and S atom are not attached directly to each other; said aryl and heterocyclic R⁴ groups are optionally fused to a C₆-C₁₀ aryl group, a C₅-C₈ saturated cyclic group, or a 5-10 membered heterocyclic group; 1 or 2 carbon atoms in the foregoing heterocyclic moieties are optionally substituted by an oxo (=O) moiety; and the alkyl, aryl and heterocyclic moieties of the foregoing R⁴ groups are optionally substituted by 1 to 3 substituents independently selected from halo, cyano, nitro, trifluoromethyl, trifluoromethoxy, azido, -NR⁶SO₂R⁵, -SO₂NR⁵R⁶, -C(O)R⁵, -C(O)OR⁵, -OC(O)R⁵, -NR⁶C₍O)R⁵, -C(O)NR⁵R⁶, -NR⁵R⁶, -(CR⁶R⁷)ₘOR⁶ wherein m is an integer from 1 to 5, -OR⁵ and the substituents listed in the definition of R⁵;
each R⁵ is independently selected from H, C₁-C₁₀ alkyl, -(CH₂)ₜ(C₆-C₁₀ aryl), and -(CH₂)ₜ(5-10 membered heterocyclic), wherein t is an integer from 0 to 5; said alkyl group optionally includes 1 or 2 hetero moieties selected from O, S and -N(R⁶)- with the proviso that two O atoms, two S atoms, or an O and S atom are not attached directly to each other; said aryl and heterocyclic R⁵ groups are optionally fused to a C₆-C₁₀ aryl group, a C₅-C₈ saturated cyclic group, or a 5-10 membered heterocyclic group; and the foregoing R⁵ substituents, except H, are optionally substituted by 1 to 3 substituents independently selected from halo, cyano, nitro, trifluoromethyl, trifluoromethoxy, azido, -C(O)R⁶, -C(O)OR⁶, -CO(O)R⁶, -NR⁶C(O)R⁷, - C(O)NR⁶R⁷, -NR⁶R⁷, hydroxy, C₁-C₆ alkyl, and C₁-C₆ alkoxy; and,
each R⁶ and R⁷ is independently H or C₁-C₆ alky;
in the manufacture of an agent for simultaneous or sequential administration for treating hyperproliferative disordersl.

Compounds of the formula 1 and their pharmaceutically acceptable salts and solvates may be prepared as described in U.S. Patent No. 6,235,764.

Anti-metabolite nucleosides and nucleoside analogs have found widespread use in the treatment of cancer and other human diseases. One such nucleoside analog, 5-fluorouracil (5-. FU) has been used continuously since its development in 1957 by Duusinski and Heidelberger (U.S. Pat. No. 2,802,005) for the treatment of solid tumors of the head and neck, breast, and colon. 5-FU was originally designed to work as an inhibitor of thymidylate synthetase (TS), the enzyme which converts deoxyuridine 5'-O-monophosphate (dUMP) to deoxythymidine 5'-O-monophosphate (dTMP). It is believed that 5-FU retards tumor expansion by causing thymidine pools to become depleted in rapidly proliferating tumor cells. Other nucleoside analogs such as gemcitabine hydrochloride are known and are a preferred compound for use in the methods and pharmaceutical compositions of the present invention.

Combinations of the invention may be administered sequentially or may be administered simultaneously.

The combination of a compound of formula 1 and gemcitabine hydrochloride is synergistic or exhibits a synergism. Synergism or synergistic as used to describe the compositions and methods of the present invention means a greater than additive biological effect. Thus, to state that cytotoxic (i.e., the taxane derivative, the platinum coordination complex, the nucleoside analog, or the anthracycline) is synergistic with compound of formula 1 means that the combination, in any form, produces greater cytotoxicity that either drug alone. In a preferred embodiment, the combinations of the present invention have a synergy of greater than 2 fold compared to each compound administered alone. In a more preferred embodiment, the combinations of the present invention have a synergy of greater than 4 fold compared to each compound administered alone.

The compounds of the present invention may have asymmetric carbon atoms. Such diasteromeric mixtures can be separated into their individual diastereomers on the basis of their physical chemical differences by methods known to those skilled in the art, for example, by chromatography or fractional crystallization. Enantiomers can be separated by converting the enantiomeric mixtures into a diastereomeric mixture by reaction with an appropriate optically active compound (e.g., alcohol), separating the diastereomers and converting (e.g., hydrolyzing) the individual diastereomers to the corresponding pure enantiomers. All such isomers, including diastereomer mixtures and pure enantiomers are considered as part of the invention.

The compounds of formula 1 that are basic in nature are capable of forming a wide variety of different salts with various inorganic and organic acids. Although such salts must be pharmaceutically acceptable for administration to animals, it is often desirable in practice to initially isolate the compound of formula 1 from the reaction mixture as a pharmaceutically unacceptable salt and then simply convert the latter back to the free base compound by treatment with an alkaline reagent and subsequently convert the latter free base to a pharmaceutically acceptable acid addition salt. The acid addition salts of the base compounds of this invention are readily prepared by treating the base compound with a substantially equivalent amount of the chosen mineral or organic acid in an aqueous solvent medium or in a suitable organic solvent, such as methanol or ethanol. Upon careful evaporation of the solvent, the desired solid salt is readily obtained. The desired acid salt can also be precipitated from a solution of the free base in an organic solvent by adding to the solution an appropriate mineral or organic acid.

Those compounds of formula 1 that are acidic in nature, are capable of forming base salts with various pharmacologically acceptable cations. Examples of such salts include the alkali metal or alkaline-earth metal salts and particularly, the sodium and potassium salts. These salts are all prepared by conventional techniques. The chemical bases which are used as reagents to prepare the pharmaceutically acceptable base salts of this invention are those which form non-toxic base salts with the acidic compounds of formulas 1. Such non-toxic base salts include those derived from such pharmacologically acceptable cations as sodium, potassium, calcium and magnesium, etc. These salts can easily be prepared by treating the corresponding acidic compounds with an aqueous solution containing the desired pharmacologically acceptable cations, and then evaporating the resulting solution to dryness, preferably under reduced pressure. Alternatively, they may also be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum yields of the desired final product.

Included in the present invention are compounds identical to the compounds of formula 1 but for the fact that one or more hydrogen or carbon atoms are replaced by isotopes thereof. Such compounds are useful as research and diagnostic tools in metabolism pharmokinetic studies and in binding assays. Specific applications in research include radioligand binding assays, autoradiography studies and in vivo binding studies. Included among the radiolabelled forms of the compounds of formula 1 are the tritium and C¹⁴ isotopes thereof.

Administration of the compounds of the present invention (hereinafter the "active compound(s)") can be effected by any method that enables delivery of the compounds to the site of action. These methods include oral routes, intraduodenal routes, parenteral injection (including intravenous, subcutaneous, intramuscular, intravascular or infusion), intraocular, intraperitoneal, intravesicular, intravaginal, topical, and rectal administration.

The amount of each of the active compounds administered will be dependent on the subject being treated, the severity of the disorder or condition, the rate of administration and the judgement of the prescribing physician. However, an effective dosage is in the range of about 0.001 to about 100 mg per kg body weight per day, preferably about 1 to about 35 mg/kg/day, in single or divided doses. For a 70 kg human, this would amount to about 0.05 to about 7 g/day, preferably about 0.2 to about 2.5 g/day. In some instances, dosage levels below the lower limit of the aforesaid range may be more than adequate, while in other cases still larger doses may be employed without causing any harmful side effect, provided that such larger doses are first divided into several small doses for administration throughout the day.

The products of which the combination are composed may be administered simultaneously, separately or spaced out over a period of time so as to obtain the maximum efficacy of the combination; it being possible for each administration to vary in its duration from a rapid administration to a continuous perfusion. As a result, for the purposes of the present invention, the combinations are not exclusively limited to those which are obtained by physical association of the constituents, but also to those which permit a separate administration, which can be simultaneous or spaced out over a period of time. The compositions according to the invention are preferably compositions which can be administered parentally. However, these compositions may be administered orally or intraperitoneally in the case of localized regional therapies.

The compositions for parental administration are generally pharmaceutically acceptable, sterile solutions or suspensions which may optionally be prepared as required at the time of use. For the preparation of non-aqueous solutions or suspensions, natural vegetable oils such as-olive oil, sesame oil or liquid petroleum or injectable organic esters such as ethyl oleate may be used. The sterile aqueous solutions can consist of a solution of the product in water. The aqueous solutions are suitable for intravenous administration provided the pH is appropriately adjusted and the solution is made isotonic, for example with a sufficient amount of sodium chloride or glucose. The sterilization may be carried out by heating or by any other means which does not adversely affect the composition.

The combinations may also take the form of liposomes or the form of an association with carriers as cyclodextrins or polyethylene glycols. The compositions for oral or intraperitoneal administration are preferably aqueous suspensions or solutions.

The combinations of the present invention are formulated alone, howerever they may also be formulated together if desired. This facilitates the easy of use (i.e., less tablets for a patient to swallow) and patient compliance since one tablet is a desired dosage form.

The pharmaceutical composition may, for example, be in a form suitable for oral administration as a tablet, capsule, pill, powder, sustained release formulations, solution, suspension, for parenteral injection as a sterile solution, suspension or emulsion, for topical administration as an ointment or cream or for rectal administration as a suppository. The pharmaceutical composition may be in unit dosage forms suitable for single administration of precise dosages. The pharmaceutical composition will include a conventional pharmaceutical carrier or excipient and a compound according to the invention as an active ingredient. In addition, it may include other medicinal or pharmaceutical agents, carriers, adjuvants, etc.

Exemplary parenteral administration forms include solutions or suspensions of active compounds in sterile aqueous solutions, for example, aqueous propylene glycol or dextrose solutions. Such dosage forms can be suitably buffered, if desired.

Suitable pharmaceutical carriers include inert diluents or fillers, water and various organic solvents. The pharmaceutical compositions may, if desired, contain additional ingredients such as flavorings, binders, excipients and the like. Thus for oral administration, tablets containing various excipients, such as citric acid may be employed together with various disintegrants such as starch, alginic acid and certain complex silicates and with binding agents such as sucrose, gelatin and acacia. Additionally, lubricating agents such as magnesium stearate, sodium lauryl sulfate and talc are often useful for tableting purposes. Solid compositions of a similar type may also be employed in soft and hard filled gelatin capsules. Preferred materials, therefore, include lactose or milk sugar and high molecular weight polyethylene glycols. When aqueous suspensions or elixirs are desired for oral administration the active compound therein may be combined with various sweetening or flavoring agents, coloring matters or dyes and, if desired, emulsifying agents or suspending agents, together with diluents such as water, ethanol, propylene glycol, glycerin, or combinations thereof.

Methods of preparing various pharmaceutical compositions with a specific amount of active compound are known, or will be apparent, to those skilled in this art. For examples, see Remington's Pharmaceutical Sciences, Mack Publishing Company, Easter, Pa., 15th Edition (1975).

The examples and preparations provided below further illustrate and exemplify the compounds of the present invention and methods of preparing such compounds. The following abbreviations are used in the Examples and have the definitions indicated unless otherwise noted: QD is once a day; BID is twice a day; Q3d X 4 is once every 3 days for a total of 4 treatments; FDS is fetal bovine serum; ul is microliter; pen/strep is penicillin/streptomycin; s.c. is subcutaneous; and po is by mouth.

### Example 1

### Anti-Tumor Efficacy of Gemcitabine Hydrochloride and mesylate salt of 3-(4-Bromo-2,6-difluoro-benzyloxy)-5-[3-(4-pyrrolidin-1-yl-butyl)-ureido)-isothiazole-4-carboxylic acid amide Against the Human Pancreatic Carcinoma Capan-1

Exponentially growing Capan-1 (RPMI 1640 with 10% FBS, and pen /strep (Gibco) were harvested and inoculated s.c. (10⁷ cells/mouse, 200 µl) into the right flank of female Nu/Nu mice (∼ 20 grams; Charles River Laboratories, MA). 7 days after inoculation, animals with tumor approximately 150 mm³ in size were separated into groups of 11 groups of 10 animals each. Gemcitabine hydrochloride (Gemzar^{®}) (Eli Lilly and Company, Indianapolis, Ind.) was formulated in 0.9% saline and compound X (the mesylate salt of 3-(4-Bromo-2,6-difluoro-benzyloxy)-5-(3-(4-pyrrolidin-1-yl-butyl)-ureido}-isothiazole-4-carboxylic acid amide) was formulated in 5% Gelucire (Gattefosse Inc., France).

An overview of each of the groups and the treatment is set forth below in the table.

| **Group #** | **Compound(s)** | **Treatment Dosage and Administration Procedure** |
|---|---|---|
| 1 | Vehicle | 5% Gelucire, po, qd x 13 |
| 2 | Compound X | 25 mg/kg, po, qd x 13 |
| 3 | Compound X | 100 mg/kg, po, qd x 13 |
| 4 | Gemcitabine HCl | 1 mg/kg, ip, q3d x 4 |
| 5 | Gemcitabine HCl | 5 mg/kg, ip, q3d x 4 |
| 6 | Gemcitabine HCl | 80 mg/kg, ip, q3d x 4 |
| 7 | Gemcitabine HCl and Compound X | Gemcitabine HCl 1 mg/kg, ip, q3d x 4 + Compound X 25 mg/kg, po, qd x 13 |
| 8 | Gemcitabine HCl and Compound X | Gemcitabine HCl 1 mg/kg, ip, q3d x 4 + Compound X, 100 mg/kg, po, qd x 13 |
| 9 | Gemcitabine HCl and Compound X | Gemcitabine HCl 5 mg/kg, ip, q3d x 4 + Compound X, 25 mg/kg, po, qd x 13 |
| 10 | Gemcitabine HCl and Compound X | Gemcitabine HCl 5 mg/kg, ip, q3d x 4 + Compound X, 100 mg/kg, po, qd x 13 |
| 11 | Gemcitabine HCl and Compound X | Gemcitabine HCl 80 mg/kg, ip, q3d x 4 + Compound X, 100 mg/kg, po, qd x 13 |

Animal body weight and tumor measurements were obtained at regular intervals. Tumor volume (mm³) was calculated using the formula length (mm) x width (mm) x width (mm) x 0.5. The following table shows the percentage (%) inhibition of tumor growth for each of the treatments.

| **Group #** | **% Tumor Growth Inhibition** |
|---|---|
| 1 | 0 |
| 2 | 16 |
| 3 | 46 |
| 4 | 0 |
| 5 | 26 |
| 6 | 83 |
| | 66% regression* |
| 7 | 86 |
| | 69% regression* |
| 8 | 96 |
| | 89% regression* |
| 9 | 67 |
| | 16% regression* |
| 10 | 88 |
| | 60% regression* |
| 11 | 91 |
| | 67% regression* |

| | |
|---|---|
| * Mean tumor volume on day 1 of individual group was used as 100 % for the calculation of mean tumor regression. | |

Mean tumor volume in Gelucire vehicle group on day 13 was used for % growth inhibition calculation.

## Claims

1. Use of (i) a therapeutically effective amount of gemcitabine hydrochloride and (ii) a therapeutically effective amount of a compound of the formula 1 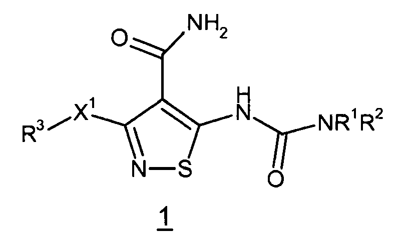 or a pharmaceutically acceptable salt or solvate thereof, wherein:
wherein X¹ is O or S;
R¹ is H, C₁-C₁₀ alkyl, C₂-C₁₀ alkenyl, C₂-C₁₀ alkynyl, -C(O)(C₁-C₁₀ alkyl), -(CH₂)ₜ(C₆-C₁₀ aryl), -(CH₂)ₜ(4-10 membered heterocyclic), -C(O)(CH₂)ₜC₆-C₁₀ aryl), or -C(O)(CH₂)ₜ(5-10 membered heterocyclic), wherein t is an integer from 0 to 5; said alkyl group optionally includes 1 or 2 hetero moieties selected from O, S and -N(R⁶)- with the proviso that two O atoms, two S atoms, or an O and S atom are not attached directly to each other; said aryl and heterocyclic R¹ groups are optionally fused to a C₆-C₁₀ aryl group, a C₅-C₈ saturated cyclic group, or a 5-10 membered heterocyclic group; 1 or 2 carbon atoms in the foregoing heterocyclic moieties are optionally substituted by an oxo (=O) moiety; the -(CH₂)ₜ- moieties of the foregoing R¹ groups optionally include a carbon-carbon double or triple bond where t is an integer from 2 to 5; and the foregoing R¹ groups, except H, are optionally substituted by 1 to 3 R⁴ groups;
R² is selected from the list of substituents provided in the definition of R¹, -SO₂(CH₂)ₜ(C₆-C₁₀ aryl), -SO₂(CH₂)ₜ(5-10 membered heterocyclic), and -OR⁵, t is an integer ranging from 0 to 5, the -(CH₂)ₜ- moieties of the foregoing R² groups optionally include a carbon-carbon double or triple bond where t is an integer from 2 to 5, and the foregoing R² groups are optionally substituted by 1 to 3 R⁴ groups;
or R¹ and R² may be taken together with the nitrogen to which each is attached to form a 4-10 membered saturated monocyclic or polycyclic ring or a 5-10 membered heteroaryl ring, wherein said saturated and heteroaryl rings optionally include 1 or 2 heteroatoms selected from O, S and -N(R⁶)- in addition to the nitrogen to which R¹ and R² are attached, said -N(R⁶)- is optionally =N- or -N= where R¹ and R² are taken together as said heteroaryl group, said saturated ring optionally may be partially unsaturated by including 1 or 2 carbon-carbon double bonds, and said saturated and heteroaryl rings, including the R⁶ group of said -N(R⁶)-, are optionally substituted by 1 to 3 R⁴ groups;
R³ is H, C₁-C₁₀ alkyl, C₂-C₁₀ alkenyl, C₂-C₁₀ alkynyl, -(CH₂)ₜC₆-C₁₀ aryl), or -(CH₂)ₜ(5-10 membered heterocyclic), wherein t is an integer from 0 to 5; said alkyl group optionally includes 1 or 2 hetero moieties selected from O, S and -N(R⁶)- with the proviso that two O atoms, two S atoms, or an O and S atom are not attached directly to each other; said aryl and heterocyclic R³ groups are optionally fused to a C₆-C₁₀ aryl group, a C₅-C₈ saturated cyclic group, or a 5-10 membered heterocyclic group; 1 or 2 carbon atoms in the foregoing heterocyclic moieties are optionally substituted by an oxo (=O) moiety; the -(CH₂)ₜ- moieties of the foregoing R³ groups optionally include a carbon-carbon double or triple bond where t is an integer from 2 to 5, and the foregoing R³ groups are optionally substituted by 1 to 5 R⁴ groups;
each R⁴ is independently selected from C₁-C₁₀ alkyl, C₂-C₁₀ alkenyl, C₂-C₁₀ alkynyl, halo, cyano, nitro, trifluoromethyl, trifluoromethoxy, azido, -OR⁵, -C(O)R⁵, -C(O)OR⁵, ,-NR⁶C(O)OR⁵, -OC(O)R⁵, -NR⁶SO₂R⁵, -SO₂NR⁵R⁸, -NR⁶C(O)R⁵, -C(O)NR⁵R⁶, -NR⁵R⁶, -S(O)ⱼR⁷ wherein j is an integer ranging from 0 to 2, -SO₃H, -NR⁵(CR⁶R)ₜOR⁶, -(CH₂)ₜ(C₆-C₁₀ aryl), -SO₂(CH₂)ₜ(C₆-C₁₀ aryl), -S(CH₂)ₜ(C₆-C₁₀ aryl), -O(CH₂)ₜ(C₆-C₁₀ aryl), -(CH₂)ₜ(5-10 membered heterocyclic), and -(CR⁶R⁷)ₘOR⁶, wherein m is an integer from 1 to 5 and t is an integer from 0 to 5; said alkyl group optionally contains 1 or 2 hetero moieties selected from O, S and -N(R⁶)- with the proviso that two O atoms, two S atoms, or an O and S atom are not attached directly to each other; said aryl and heterocyclic R⁴ groups are optionally fused to a C₆-C₁₀ aryl group, a C₅-C₈ saturated cyclic group, or a 5-10 membered heterocyclic group; 1 or 2 carbon atoms in the foregoing heterocyclic moieties are optionally substituted by an oxo (=O) moiety; and the alkyl, aryl and heterocyclic moieties of the foregoing R⁴ groups are optionally substituted by 1 to 3 substituents independently selected from halo, cyano, nitro, trifluoromethyl, trifluoromethoxy, azido, -NR⁶SO₂R⁵, -SO₂NR⁵R⁶, -C(O)R⁵, -C(O)OR⁵, -OC(O)R⁵, -NR⁶C(O)R⁵, -C(O)NR⁵R⁶, -NR⁵R⁶, -(CR⁶R⁷)ₘOR⁶ wherein m is an integer from 1 to 5, -OR⁵ and the substituents listed in the definition of R⁵
each R⁵ is independently selected from H, C₁-C₁₀ alkyl, -(CH₂)ₜ(C₆-C₁₀ aryl), and .-(CH₂)ₜ(5-10 membered heterocyclic), wherein t is an integer from 0 to 5; said alkyl group optionally includes 1 or 2 hetero moieties selected from O, S and -N(R⁶)- with the proviso that two O atoms, two S atoms, or an O and S atom are not attached directly to each other; said aryl and heterocyclic R⁵ groups are optionally fused to a C₆-C₁₀ aryl group, a C₅-C₈ saturated cyclic group, or a 5-10 membered heterocyclic group; and the foregoing R⁵ substituents, except H, are optionally substituted by 1 to 3 substituents independently selected from halo, cyano, nitro, trifluoromethyl, trifluoromethoxy, azido, -C(O)R⁶, -C(O)OR⁶, -CO(O)R⁶, -NR⁶C(O)R⁷, - C(O)NR⁶R⁷, -NR⁶R⁷, hydroxy, C₁-C₆ alkyl, and C₁-C₆ alkoxy; and
each R⁶ and R⁷ is independently H or C₁-C₆ alkyl;
for the manufacturing of pharmaceuticals for simultaneous or sequential administration for treating hyperproliferative disorders.

2. The use of claim 1, wherein the hyperproliferative disorder is cancer.

3. The use of claim 2, wherein said cancer is selected from the group consisting of brain, squamous cell, bladder, gastric, pancreatic, breast, head, neck, oesophageal, prostate, colorectal, lung, renal, kidney, ovarian, gynecological and thyroid cancer.

4. The use of any one of claims 1 to 3, wherein said compounds (i) and (ii) are administered simultaneously.

5. The use of any one of claims 1 to 3, wherein said compounds (i) and (ii) are administered sequentially.

6. The use of any one of claims 1 to 5, wherein R² of the compound of formula 1 is H and R¹ is C₁-C₁₀ alkyl optionally substituted by 1 or 2 substituents independently selected from - NR⁵R⁶, -NR⁵(CR⁶R⁷)ₜOR⁶ and -(CH₂)ₜ(5-10 membered heterocyclic) wherein t is an integer from 0 to 5.

7. The use of any one of claims 1 to 5, wherein R² of the compound of formula 1 is H and R¹ of the compound of formula 1 is -(CH₂)ₜ(5-10 membered heterocyclic), wherein t is an integer from 0 to 5; said heterocyclic group is optionally fused to a C₆-C₁₀ aryl group, a C₅-C₈ saturated cyclic group, or a 5-10 membered heterocyclic group; and said R¹ group, including the optionally fused portions of said R¹ group, is optionally substituted by 1 or 2 substituents independently selected from C₁-C₄ alkyl, hydroxy and hydroxymethyl.

8. The use according to any one of claims 1 to 5, wherein R³ of the compound of formula 1 is -(CH₂)ₜ(C₆-C₁₀ aryl) wherein t is an integer from 1 to 3 and said R³ group is optionally substituted by 1 to 4 R⁴ groups.

9. The use according to any one of claims 1 to 5, wherein the compound of formula 1 is selected from the group consisting of
mesylate salt of 3-(4-Bromo-2,6-difluoro-benzyloxy)-5-[3-(4-pyrrolidin-1-yl-butyl)-ureido}-isothiazole-4-carboxylic acid amide;
5-{3-[3-(4-Methyl-piperazin-1-yl)-propyl]-ureido}-3-(2,3,6-trifluoro-4-methyl-benzyloxy)-isothiazole-4-carboxylic acid amide;
3-(4-Chloro-2,6-difluoro-benzyloxy)-5-(3-{4-[ethyl-(2-hydroxy-ethyl)-amino]-butyl}-ureido)-isothiazole-4-carboxylic acid amide;
3-(2-Fluoro-4-methyl-benzyloxy)-5-{3-[3-(4-methyl-piperazin-1-yl)-propyl]-ureido}-isothiazole-4-carboxylic acid amide;
3-(2,5-Difluoro-4-methyl-benzyloxy)-5-(3-4-[4-(2-hydroxy-ethyl)-piperazin-1-yl]-butyl}-ureido)-isothiazole-4-carboxylic acid amide;
3-(2,5-Difluoro-4-methyl-benzyloxy)-5-[3-(6-dimethylamino-hexyl)-ureido]-isothiazole-4-carboxylic acid amide;
3-(2-Fluoro-4-methyl-benzyloxy)-5-[3-(5-isopropylamino-pentyl)-ureido]-isothiazole-4-carboxylic acid amide;
hydrochloride salt of 3-(4-Bromo-2,6-difluoro-benzyloxy)-5-[3-(4-pyrrolidin-1-yl-butyl)-ureido}-isothiazole-4-carboxylic acid amide;
3-(4-Chloro-2,6-difluoro-benzyloxy)-5-[3-(4-pyrrolidin-1-yl-butyl)-ureido]-isothiazole-4-carboxylic acid amide;
3-(4-Chloro-2,6-difluoro-benzyloxy)-5-{3-[3-(4-methyl-piperazin-1-yl)-propyl]-ureido}-isothiazole-4-carboxylic acid amide;
3-(4-Chloro-2,6-difluoro-benzyloxy)-5-{3-[-(1-methyl-pyrrolidin-2-yl)-ethyl]-ureido}-isothiazole-4-carboxylic acid amide;
3-(2,6-Difluoro-4-methyt-benzyloxy)-5-[3-(4-pyrrolidin-1-yl-butyl)-ureido]-isothiazole-4-carboxylic acid amide;
3-(2,6-Difluoro-4-methyl-benzyloxy)-5-[3-{4-[4-(2-hydroxy-ethyl)-piperazin-1-yl]-butyl}-ureido)-isothiazole-4-carboxylic acid amide;
3-(4-Chloro-2,6-difluoro-benzyloxy)-5-[3-(3-hydroxy-5-pyrrolidin-1-yl)-pentyl)-ureido}-isothiazole-4-carboxylic acid amide;
3-(2,5-Difluoro-4-methyl-benzyloxy)-5-{3-[4-(3,4-dihydroxy-pyrrolidin-1-yl]-butyl]-ureido}-isothiazole-4-carboxylic acid amide;
3-(4-Chloro-2,6-difluoro-benzyloxy)-5-(3-[4-(3,4-dihydroxy-pyrrolidin-1-yl)-butyl]-ureido)-isothiazole-4-carboxylic acid amide;
3-(2,5-Difluoro-4-methyl-benzyloxy)-5-{3-[4-(2-hydroxymethyl-pyrrolidin-1-yl)-butyl]-ureido}-isothiazole-4-carboxylic acid amide;
3-(4-Chloro-2,6-difluoro-benzyloxy)-5-{3-[4-(2-hydroxymethyl-pyrrolidin-1-yl)-butyl]-ureido}-isothiazole-4-carboxylic acid amide;
3-(2,5-Difluoro-4-methyl-benzyloxy)-5-{3-[4-(3-hydroxy-pyrrolidin-1-yl)-butyl]-ureido}-isothiazole-4-carboxylic acid amide;
3-(4-Bromo-2,6-difluoro-benzyloxy)-5-[3-(4-pyrrolidin-1-yl-butyl]-ureido}-isothiazole-4-carboxylic acid amide;
3-(2,6-Difluoro-4-methyl-benzyloxy)-5-[3-(4-hydroxy-5-piperidin-1-yl-pentyl)-ureido]-isothiazole-4-carboxylic acid amide;
3-(2,5-Difluoro-4-methyl-benzyloxy)-5-{3-[4-(3-hydroxy-5-piperidin-1-yl-pentyl)-ureido]-isothiazole-4-carboxylic acid amide;
3-(4-Chloro-2,6-difluoro-benzyloxy)-5-{3-[4-(2-hydroxymethyl-piperidin-1-yl)-butyl]-ureido}-isothiazole-4-carboxylic acid amide;
3-(2,5-Difluoro-4-methyl-benzyloxy)-5-(3-{4-[ethyl-(2-hydroxy-ethyl)-amino]-butyl}-ureido)-isothiazole-4-carboxylic acid amide;
3-(4-Chloro-2,6-difluoro-benzyloxy)-5-[3-(5-hydroxy-6-piperidin-1-yl)-hexyl)-ureido}-isothiazole-4-carboxylic acid amide;
3-(4-Bromo-2,3,6-trifluoro-benzyloxy)-5-(3-[3-(4-methyl-piperazin-1-yl)-propyl]-ureido)-isothiazole-4-carboxylic acid amide;
3-(2,6-Difluoro-4-methyl-benzyloxy)-5-{3-[3-(4-methyl-piperazin-1-yl-propyl]-ureido}-isothiazole-4-carboxylic acid amide;
3-(2,6-Difluoro-4-methyl-benzyloxy)-5-[3-(3-hydroxy-5-pyrrolidin-1-yl-pentyl)-ureido]-isothiazole-4-carboxylic acid amide;
5-[3-(4-Pyrrolidin-1-yl-butyl)-ureido]-3-(2,3,6-trifluoro-4-methyl-benzyloxy)-isothiazole-4-carboxylic acid amide;
5-[3-(3-Hydroxy-5-pyrrolidin-1-yl-pentyl)-ureido]-3-(2,3,6-trifluoro-4-methyl-benzyloxy)-isothiazole-4-carboxylic acid amide;
3-(4-Chloro-2,6-difluoro-benzyloxy)-5-{3-[3-(5-methyl-2,5-diazabicyclo[2.2.1] hept-2-yl)-propyl]-ureido}-isothiazole-4-carboxylic acid amide;
3-(4-Chloro-2,3,6-trifluoro-benzyloxy)-5-{3-[3-(5-methyl-2,5-diaza-bicyclo[2.2.1]hept-2-yl)-propyl]-ureido}-isothiazole-4-carboxylic acid amide;
3-(4-Chloro-2,3,6-trifluoro-benzyloxy)-5-{3-[2-(1-methyl-pyrrolidin-2-yl)-ethyl]-ureido}-isothiazole-4-carboxylic acid amide;
3-(4-Chloro-2,3,6-trifluoro-benzyloxy)-5-[3-(4-pyrrolidin-1-yl-butyl)-ureido]-isothiazole-4-carboxylic acid amide;
3-(4-Chloro-2,3,6-trifluoro-benzyloxy)-5-{3-[4-(2-hydroxymethyl-pyrrolidin-1-yl)-butyl]-ureido}-isothiazole-4-carboxylic acid amide;
5-{3-[2-(1-Methyl-pyrrolidin-2-yl)-ethyl]-ureido}-3-(2,3,6-trifluoro-4-methyl-benzyloxy)-isothiazole-4-carboxylic acid amide;
5-[3-(4-Dimethylamino-butyl)-ureido]-3-(2,3,6-trifluoro-4-methyl-benzyloxy)-isothiazole-4-carboxylic acid amide;
5-[3-(3-Dimethylamino-propyl)-ureido]-3-(2,3,6-trifluoro-4-methyl-benzyloxy)-isothiazole-4-carboxylic acid amide;
5-[3-(3-Hydroxy-5-isopropropylamino-pentyl)-ureido]-3-(2,3,6-trifluoro-4-methyl-benzyloxy)-isothiazole-4-carboxylic acid amide;
5-[3-(3-Isopropylamino-propyl)-ureido]-3-(2,3,6-trifluoro-4-methyl-benzyloxy)-isothiazole-4-carboxylic acid amide;
5-{3-[4-(4-Methyl-piperazin-1-yl)-butyl]-ureido}-3-(2,3,6-trifluoro-4-methyl-benzyloxy)-isothiazole-4-carboxylic acid amide;
5-(3-{4-[4-(2-Hydroxy-ethyl)-piperazin-1-yl]-butyl}-ureido)-3-(2,3,6-trifluoro-4-methyl-benzyloxy)-isothiazole-4-carboxylic acid amide;
5-[3-(3-Pyrrolidin-1-yl-propyl)-ureido]-3-(2,3,6-trifluoro-4-methyl-benzyloxy)-isothiazole-4-carboxylic acid amide;
5-[3-(4-Hydroxy-5-piperidin-1-yl-pentyl)-ureido]-3-(2,3,6-trifluoro-4-methyl-benzyloxy)-isothiazole-4-carboxylic acid amide;
3-(4Chloro-2,6-difluoro-benzyloxy)-5-[3-(4-imidazol-1-yl-butyl)-ureido]-isothiazole-4-carboxylic acid amide;
5-(3-{4-[Ethyl-(2-hydroxy-ethyl}-amino]-butyl}-ureido)-3-(2,3,6-trifluoro-4-methyl-benzyloxy)-isothiazole-4-carboxylic acid amide;
3-(4-Chloro-(2,3,6-trifluoro-benzyloxy)-5-{3-[4-(2-hydroxmethyl-piperidin-1-yl)-butyl]-ureido}-isothiazole-4-carboxylic acid amide;
3-(4-Chloro-2,3,6-trifluoro-benzyloxy)-5-[3-(3-hydroxy-5-pyrrolidin-1-yl-pentyl)-ureido]-isothiazole-4-carboxylic acid amide;
3-(4-Bromo-2,6-difluoro-benzyloxy)-5-{3-[3-(4-methyl-piperazin-1-yl)-propyl]-ureido}-isothiazole-4-carboxylic acid amide;
3-(2,6-Difluoro-4-methyl-benzyloxy)-5-{3-[2-(1-methyl-pyrrolidin-2-yl)-ethyl]-ureido}-isothiazole-4-carboxylic acid amide;
3-(2,6-Difluoro-4-methyl-benzyloxy)-5-[3-(4-dimethylamino-butyl)-ureido}-isothiazole-4-carboxylic acid amide;
3-(2,6-Difluoro-4-methyl-benzyloxy)-5-[3-(3-dimethylamino-propyl)-ureido]-isothiazole-4-carboxylic acid amide;
3-(4-Bromo-2,3,6-trifluoro-benzyloxy)-5-[3-(4-pyrrolidin-1-yl-butyl)-ureido]-isothiazole-4-carboxylic acid amide;
3-(4-Chloro-2,3,6-trifluoro-benzyloxy)-5-[3-(4-imidazol-1-yl-butyl)-ureido]-isothiazole-4-carboxylic acid amide;
3-(4-Chloro-2,3,6-difluoro-benzyloxy)-5-(3-{3-[ethyl-(2-hydroxy-ethyl)-amino]-propyl}-ureido)-isothiazole-4-carboxylic acid amide;
3-(4-Chloro-2,3,6-trifluoro-benzyloxy)-5-(3-{3-[ethyl-(2-hydroxy-ethyl)-amino]-propyl}-ureido)-isothiazole-4-carboxylic acid amide;
5-[3-(3-Methylamino-propyl)-ureido]-3-(2,3,6-trifluoro-4-methyl-benzyloxy)-isothiazole-4-carboxylic acid amide;
5-[3-(3-Amino-propyl)-3-methyl-ureido]-3-(2,3,6-trifluoro-4-methyl-benzyloxy)-isothiazole-4-carboxylic acid amide;
5-[3-(4-Diethylamino-butyl)-ureido]-3-(2,3,6-trifluoro-4-methyl-benzyloxy)-isothiazole-4-carboxylic acid amide;
3-(2,6-Difluoro-4-methyl-benzyloxy)-5-[3-(3-pyrrolidin-1-yl-propyl)-ureido]-isothiazole-4-carboxylic acid amide;
3-(3-Chloro-2,6-difluoro-4-methyl-benzyloxy)-5-[3-(4-dimethylamino-butyl)-ureido]-isothiazole-4-carboxylic acid amide;
5-(3-{4-[Bis-(2-hydroxy-ethyl)-amino]-butyl}-ureido)-3-(2,6-difluoro-4-methyl-benzyloxy)-isothiazole-4-carboxylic acid amide;
and the pharmaceutically acceptable salts, prodrugs and solvates of said compounds.

10. The use according to claim 1, wherein the compound of formula 1 is the hydrochloride salt of 3-(4-Bromo-2,6-difluoro-benzyloxy)-5-[3-(4-pyrrolidin-1-yl-butyl)-ureido]-isothiazole-4-carboxylic acid amide).

11. A pharmaceutical composition for use in the treatment of a hyperproliferative disorder in a mammal which comprises (i) gemcitabine hydrochloride and (ii) a therapeutically effective amount of a compound of the formula 1, as defined in any one of claims 1 or 6-10, in combination with one or more pharmaceutical carriers or vehicles.

12. A kit comprising in a first compartment a compound of formula 1 as defined in any one of claims 1 or 6-10, and in a second compartment gemcitabine hydrochloride.

13. Gemcitabine hydrochloride for use in the treatment of hyperproliferative disorders in combination with a compound of formula 1, as defined in any one of claims 1 or 6-10, or a pharmaceutically acceptable salt or solvate thereof.

14. A compound of formula 1, as defined in any one of claims 1 or 6-10, or a pharmaceutically acceptable salt or solvate thereof for use in the treatment of hyperproliferative disorders in combination with gemcitabine hydrochloride.

15. A compound of formula 1, as defined in any one of claims 1 or 6-10, or a pharmaceutically acceptable salt or solvate thereof in combination with gemcitabine hydrochloride for use in the treatment of hyperproliferative disorders.

## Patentansprüche

1. Verwendung (i) einer therapeutisch wirksamen Menge von Gemcitabin-hydrochlorid und (ii) einer therapeutisch wirksamen Menge einer Verbindung der Formel 1 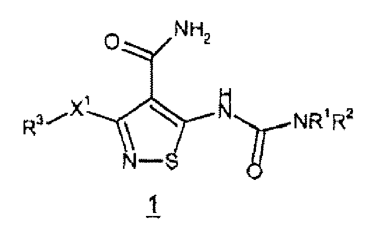 oder eines pharmazeutisch unbedenklichen Salzes oder Solvats davon, worin:
X¹ für 0 oder S steht;
R¹ für H , C₁-C₁₀-Alkyl, C₂-C₁₀-Alkenyl, C₂-C₁₀-Alkinyl, -C (0) (C₁-C₁₀-Alkyl) - (CH₂)ₜ (C₆-C₁₀-Aryl) , -(CH₂)ₜ₍4-10-gliedriger Heterocyclus), -C (O) (CH₂) ₜ (C₆-C₁₀-Aryl) oder -C (O) (CH₂) ₜ (5-10-gliedriger Heterocyclus) steht, worin t für eine ganze Zahl von 0 bis 5 steht; die Alkylgruppe gegebenenfalls 1 oder 2 unter 0, S und -N(R⁶)-ausgewählte Heterogruppierungen enthält, mit der Maßgabe, daß zwei O-Atome, zwei S-Atome oder ein 0- und ein S-Atom nicht direkt aneinander gebunden sind; die Aryl- und Heterocyclusgruppen R¹ gegebenenfalls mit einer C₆-C₁₀-Arylgruppe, einer gesättigten cyclischen C₅-C₈-Gruppe oder einer 5-10-gliedrigen heterocyclischen Gruppe anelliert sind; 1 oder 2 Kohlenstoffatome in den vorstehenden heterocyclischen Gruppierungen gegebenenfalls durch eine Oxogruppierung (=0) substituiert sind; die -(CH₂)ₜ-Gruppierungen der vorstehenden Gruppen R¹ gegebenenfalls eine Kohlenstoff-Kohlenstoff-Doppelbindung oder -Dreifachbindung enthalten, wobei t für eine ganze Zahl von 2 bis 5 steht; und die vorstehenden Gruppen R¹ außer H gegebenenfalls durch 1 bis 3 Gruppen R⁴ substituiert sind;
R² aus der Liste der in der Definition von R¹ bereitgestellten Substituenten, -SO₂(CH₂)ₜ(C₆-C₁₀-Aryl), -SO₂(CH₂)ₜ(5-10-gliedriger Heterocyclus) und -OR⁵ ausgewählt ist, t für eine ganze Zahl von 0 bis 5 steht , die -(CH₂)ₜ-Gruppierungen der vorstehenden Gruppen R² gegebenenfalls eine Kohlenstoff-Kohlenstoff-Doppelbindung oder - Dreifachbindung enthalten, wobei t für eine ganze Zahl von 2 bis 5 steht; und die vorstehenden Gruppen R² gegebenenfalls durch 1 bis 3 Gruppen R⁴ substituiert sind;
oder R¹ und R² zusammen mit dem Stickstoff, an den sie gebunden sind, einen 4-10-gliedrigen gesättigten monocylischen oder polycyclischen Ring oder einen 5-10-gliedrigen Heteroarylring bilden können, wobei die gesättigten Ringe und Heteroarylringe neben dem Stickstoff, an den R¹ und R² gebunden sind, gegebenenfalls 1 oder 2 unter 0, S und -N(R⁶)- ausgewählte Heteroatome enthalten können, das -N(R⁶)- gegebenenfalls für =N- oder -N= steht, wobei R¹ und R² als die Heteroarylgruppe zusammengenommen sind, der gesättigte Ring gegebenenfalls teilweise ungesättigt sein kann, indem er 1 oder 2 Kohlenstoff-Kohlenstoff-Doppelbindungen enthält, und die gesättigten Ringe und Heteroarylringe einschließlich der Gruppe R⁶ von -N(R⁶)- gegebenenfalls durch 1 bis 3 Gruppen R⁴ substituiert sind;
R³ für H, C₁-C₁₀-Alkyl, C₂-C₁₀-Alkenyl, C₂-C₁₀-Alkinyl, -(CH₂)ₜ(C₀-C₁₀-Aryl) oder - (CH₂)ₜ(5-10-gliedriger Heterocyclus) steht, worin t für eine ganze Zahl von 0 bis 5 steht; die Alkylgruppe gegebenenfalls 1 oder 2 unter 0, S und -N(R⁶)-ausgewählte Heterogruppierungen enthält, mit der Maßgabe, daß zwei O-Atome, zwei S-Atome oder ein 0- und ein S-Atom nicht direkt aneinander gebunden sind; die Aryl- und Heterocyclusgruppen R³ gegebenenfalls mit einer C₆-C₁₀-Arylgruppe, einer gesättigten cyclischen C₅-C₈-Gruppe oder einer 5-10-gliedrigen heterocyclischen Gruppe anelliert sind; 1 oder 2 Kohlenstoffatome in den vorstehenden heterocyclischen Gruppierungen gegebenenfalls durch eine Oxogruppierung (=0) substituiert sind;
die -(CH₂)ₜ-Gruppierungen der vorstehenden Gruppen R³ gegebenenfalls eine Kohlenstoff-Kohlenstoff-Doppelbindung oder -Dreifachbindung enthalten, wobei t für eine ganze Zahl von 2 bis 5 steht; und
die vorstehenden Gruppen R³ gegebenenfalls durch 1 bis 5 Gruppen R⁴ substituiert sind;
R⁴ jeweils unabhängig voneinander unter C₁-C₁₀-Alkyl, C₂-C₁₀-Alkenyl, C₂-C₁₀-Alkinyl, Halogen, Cyano, Nitro, Trifluormethyl, Trifluormethoxy, Azido, -OR⁵, -C(O)R⁵, -C(O)OR⁵, -NR⁶C(O)OR⁵, -OC(O)R⁵, -NR⁶SO₂R⁵, -SO₂NR⁵R⁶, -NR⁶C(O)R⁵, -C(O)NR⁵R⁶, -NR⁵R⁶, -S(O)ⱼR⁷, worin j für eine ganze Zahl im Bereich von 0 bis 2 steht, -SO₃H, -NR⁶(CR⁶R⁷)ₜOR⁶, - (CH₂)ₜ(C₀-C₁₀-Aryl), -SO₂(CH₂)ₜ(C₆-C₁₀-Aryl) , -S (CH₂)ₜ(C₆-C₁₀-Aryl) , -0 (CH₂)ₜ(C₆-C₁₀-Aryl), -(CH₂)ₜ(5-10-gliedriger Heterocyclus) und -(CR⁶R⁷)ₘOR⁶ ausgewählt ist, worin m für eine ganze Zahl von 1 bis 5 steht und t für eine ganze Zahl von 0 bis 5 steht; die Alkylgruppe gegebenenfalls 1 oder 2 unter 0, S und -N(R⁶)- ausgewählte Heterogruppierungen enthält, mit der Maßgabe, daß zwei O-Atome, zwei S-Atome oder ein 0- und ein S-Atom nicht direkt aneinander gebunden sind; die Aryl- und Heterocyclusgruppen R⁴ gegebenenfalls mit e i n e r C₆-C₁₀-Arylgruppe, einer gesättigten cyclischen C₅-C₈-Gruppe oder einer 5-10-gliedrigen heterocyclischen Gruppe anelliert sind; 1 oder 2 Kohlenstoffatome in den vorstehenden heterocyclischen Gruppierungen gegebenenfalls durch eine Oxogruppierung (=0) substituiert sind; und die Alkyl-, Aryl- und Heterocyclusgruppierungen der vorstehenden Gruppen R⁴ gegebenenfalls durch 1 bis 3 unabhängig voneinander unter Halogen, Cyano, Nitro, Trifluormethyl, Trifluormethoxy, Azido, -NR⁶SO₂R⁵, -SO₂NR⁵R⁶, -C(O)R⁵, -C(O)OR⁵, -OC(O)R⁵, -NR⁶C(O)R⁵, -C(O)NR⁵R⁶, -NR⁵R⁶, -(CR⁶R⁷)ₘOR⁶, worin m für eine ganze Zahl von 1 bis 5 steht, -OR⁵ und die in der Definition von R⁵ aufgelisteten Substituenten ausgewählte Substituenten substituiert sind;
R⁵ jeweils unabhängig voneinander unter H, C₁-C₁₀-Alkyl, -(CH₂)ₜ(C₀-C₁₀-Aryl) und - (CH₂)ₜ(5-10-gliedriger Heterocyclus) ausgewählt ist, worin t für eine ganze Zahl von 0 bis 5 steht; die Alkylgruppe gegebenenfalls 1 oder 2 unter 0, S und -N(R⁶)-ausgewählte Heterogruppierungen enthält, mit der Maßgabe, daß zwei O-Atome, zwei S-Atome oder ein 0- und ein S-Atom nicht direkt aneinander gebunden sind; die Aryl- und Heterocyclusgruppen R⁵ gegebenenfalls mit einer C₆-C₁₀-Arylgruppe, einer gesättigten cyclischen C₅-C₈-Gruppe oder einer 5-10-gliedrigen heterocyclischen Gruppe anelliert sind und die vorstehenden Substituenten R⁵ außer H gegebenenfalls durch 1 bis 3 unabhängig voneinander unter Halogen, Cyano, Nitro, Trifluormethyl, Trifluormethoxy, Azido, -C(O)R⁶, -C(O)OR⁶, -CO(O)R⁶, -NR⁶C(O)R⁷, -C(O)NR⁶R⁷, -NR⁶R⁷, Hydroxy, C₁-C₆-Alkyl und C₁-C₆-Alkoxy ausgewählte Substituenten substituiert sind; und
R⁶ und R⁷ jeweils unabhängig voneinander für H oder C₁-C₆-Alkyl stehen;
zur Herstellung von Pharmazeutika zur gleichzeitigen oder aufeinanderfolgenden Verabreichung zur Behandlung von hyperproliferativen Störungen.

2. Verwendung nach Anspruch 1, wobei es sich bei der hyperproliferativen Störung um Krebs handelt.

3. Verwendung nach Anspruch 2, wobei der Krebs aus der Gruppe bestehend aus Gehirnkrebs, Plattenepithelkrebs, Blasenkrebs, Magenkrebs, Bauchspeicheldrüsenkrebs, Brustkrebs, Kopfkrebs, Halskrebs, Speiseröhrenkrebs, Prostatakrebs, Kolorektalkrebs, Lungenkrebs, Renalkrebs, Nierenkrebs, Eierstockkrebs, gynäkologischem Krebs und Schilddrüsenkrebs ausgewählt ist.

4. Verwendung nach einem der Ansprüche 1 bis 3, wobei die Verbindungen (i) und (ii) gleichzeitig verabreicht werden.

5. Verwendung nach einem der Ansprüche 1 bis 3, wobei die Verbindungen (i) und (ii) nacheinander verabreicht werden.

6. Verwendung nach einem der Ansprüche 1 bis 5, wobei R² der Verbindung der Formel 1 für H steht und R¹ für C₁-C₁₀-Alkyl, das gegebenenfalls durch 1 oder 2 unabhängig voneinander unter -NR⁵R⁶, -NR⁵(CR⁶R⁷)ₜOR⁶ und -(CH₂)ₜ(5-10-gliedriger Heterocyclus) ausgewählte Substituenten substituiert ist, steht, worin t für eine ganze Zahl von 0 bis 5 steht.

7. Verwendung nach einem der Ansprüche 1 bis 5, wobei R² der Verbindung der Formel 1 für H steht und R¹ der Verbindung der Formel 1 für - (CH₂)ₜ(5-10-gliedriger Heterocyclus) steht, worin t für eine ganze Zahl von 0 bis 5 steht; die heterocyclische Gruppe gegebenenfalls mit einer C₆-C₁₀-Arylgruppe, einer gesättigten cyclischen C₅-C₈-Gruppe oder einer 5-10-gliedrigen heterocyclischen Gruppe anelliert ist und die Gruppe R¹, einschließlich der gegebenenfalls anellierten Teile der Gruppe R¹, gegebenenfalls durch 1 oder 2 unabhängig voneinander unter C₁-C₄-Alkyl, Hydroxy und Hydroxymethyl ausgewählte Substituenten substituiert ist.

8. Verwendung nach einem der Ansprüche 1 bis 5, wobei R³ der Verbindung der Formel 1 für - (CH₂)ₜ(C₀-C₁₀-Aryl) steht, worin t für eine ganze Zahl von 1 bis 3 steht, und die Gruppe R³ gegebenenfalls durch 1 bis 4 Gruppen R⁴ substituiert ist.

9. Verwendung nach einem der Ansprüche 1 bis 5, wobei die Verbindung der Formel 1 aus der Gruppe bestehend aus
Mesylatsalz von 3-(4-Brom-2,6-difluorbenzyloxy)-5-[3-(4-pyrrolidin-1-ylbutyl)ureido}isothiazol-4-carbonsäureamid;
5-{3-[3-(4-Methylpiperazin-1-yl)propyl]ureido}-3-(2,3,6-trifluor-4-methylbenzyloxy)isothiazol-4-carbonsäureamid;
3-(4-Chlor-2,6-difluorbenzyloxy)-5-(3-{4-[ethyl-(2-hydroxyethyl)amino]butyl}ureido)isothiazol-4-carbonsäureamid;
3-(2-Fluor-4-methylbenzyloxy)-5-{3-[3-(4-methylpiperazin-1-yl)propyl]ureido}isothiazol-4-carbonsäureamid;
3-(2,5-Difluor-4-methylbenzyloxy)-5-{3-4-[4-(2-hydroxyethyl)piperazin-1-yl]butyl)ureido)iso-thiazol-4-carbonsäureamid;
3-(2,5-Difluor-4-methylbenzyloxy)-5-[3-(6-di-methylaminohexyl)ureido]isothiazol-4-carbonsäureamid;
3-(2-Fluor-4-methylbenzyloxy)-5-[3-(5-isopropylaminopentyl)ureido]isothiazol-4-carbonsäureamid;
Hydrochloridsalz von 3-(4-Brom-2,6-difluorbenzyloxy)-5-[3-(4-pyrrolidin-1-ylbutyl)ureido}iso-thiazol-4-carbonsäureamid;
3-(4-Chlor-2,6-difluorbenzyloxy)-5-[3-(4-pyrrolidin-1-ylbutyl)ureido]isothiazol-4-carbonsäureamid;
3-(4-Chlor-2,6-difluorbenzyloxy)-5-{3-[3-(4-methylpiperazin-1-yl)propyl]ureido}isothiazol-4-carbonsäureamid;
3-(4-Chlor-2,6-difluorbenzyloxy)-5-{3-[(1-methylpyrrolidin-2-yl)ethyl]ureido}isothiazol-4-carbonsäureamid;
3-(2,6-Difluor-4-methylbenzyloxy)-5-[3-(4-pyrrolidin-1-ylbutyl)ureido]isothiazol-4-carbonsäureamid;
3-(2,6-Difluor-4-methylbenzyloxy)-5-[3-{4-[4-(2-hydroxyethyl)piperazin-1-yl]butyl}ureido)isothiazol-4-carbonsäureamid;
3-(4-Chlor-2,6-difluorbenzyloxy)-5-(3-(3-hydroxy-5-pyrrolidin-1-yl)pentyl)ureido}isothiazol-4-carbonsäureamid;
3-(2,5-Difluor-4-methylbenzyloxy)-5-{3-[4-(3,4-dihydroxypyrrolidin-1-yl]butyl]ureido}isothiazol-4-carbonsäureamid;
3-(4-Chlor-2,6-difluorbenzyloxy)-5-{3-[4-(3,4-dihydroxypyrrolidin-1-yl)butyl]ureido)isothiazol-4-carbonsäureamid;
3-(2,5-Difluor-4-methylbenzyloxy)-5-{3-[4-(2-hydroxymethylpyrrolidin-1-yl)butyl]ureido}isothiazol-4-carbonsäureamid;
3-(4-Chlor-2,6-difluorbenzyloxy)-5-{3-[4-(2-hydroxymethylpyrrolidin-1-yl)butyl]ureido}isothiazol-4-carbonsäureamid;
3-(2,5-Difluor-4-methylbenzyloxy)-5-{3-[4-(3-hydroxypyrrolidin-1-yl)butyl]ureido}isothiazol-4-carbonsäureamid;
3-(4-Brom-2,6-difluorbenzyloxy)-5-[3-(4-pyrrolidin-1-ylbutyl]ureido}isothiazol-4-carbonsäureamid;
3-(2,6-Difluor-4-methylbenzyloxy)-5-[3-(4-hydroxy-5-piperidin-1-ylpentyl)ureido]isothiazol-4-carbonsäureamid;
3-(2,5-Difluor-4-methylbenzyloxy)-5-{3-[4-(3-hydroxy-5-piperidin-1-ylpentyl)ureido]isothiazol-4-carbonsäureamid;
3-(4-Chlor-2,6-difluorbenzyloxy)-5-{3-[4-(2-hydroxymethylpiperidin-1-yl)butyl]ureido}isothiazol-4-carbonsäureamid;
3-(2,5-Difluor-4-methylbenzyloxy)-5-(3-{4-[ethyl (2-hydroxy-ethyl)amino]butyl}ureido)isothiazol-4-carbonsäureamid;
3-(4-Chlor-2,6-difluorbenzyloxy)-5-(3-(5-hydroxy-6-piperidin-1-yl)hexyl)ureido}isothiazol-4-carbonsäureamid;
3-(4-Brom-2,3,6-trifluorbenzyloxy)-5-{3-[3-(4-methylpiperazin-l-yl)propyl]ureido}isothiazol-4-carbonsäureamid;
3-(2,6-Difluor-4-methylbenzyloxy)-5-{3-[3-(4-methylpiperazin-1-ylpropyl]ureido}isothiazol-4-carbonsäureamid;
3-(2,6-Difluor-4-methylbenzyloxy)-5-[3-(3-hydroxy-5-pyrrolidin-1-ylpentyl)ureido]isothiazol-4-carbonsäureamid;
5-[3-(4-Pyrrolidin-1-ylbutyl)ureido]-3-(2,3,6-trifluor-4-methylbenzyloxy)isothiazol-4-carbonsäureamid;
5-[3-(3-Hydroxy-5-pyrrolidin-1-ylpentyl)ureido]-3-(2,3,6-trifluor-4-methylbenzyloxy)isothiazol-4-carbonsäureamid;
3-(4-Chlor-2,6-difluorbenzyloxy)-5-{3-[3-(5-methyl-2,5-diazabicyclo[2.2.1]hept-2-yl)propyl]-ureido}isothiazol-4-carbonsäureamid;
3-(4-Chlor-2,3,6-trifluorbenzyloxy)-5-{3-[3-(5-methyl-2,5-diazabicyclo[2.2.1]hept-2-yl)propyl]-ureido}isothiazol-4-carbonsäureamid;
3-(4-Chlor-2,3,6-trifluorbenzyloxy)-5-{3-[2-(1-methylpyrrolidin-2-yl)ethyl]ureido}isothiazol-4-carbonsäureamid;
3-(4-Chlor-2,3,6-trifluorbenzyloxy)-5-[3-(4-pyrrolidin-1-ylbutyl)ureido]isothiazol-4-carbonsäureamid;
3-(4-Chlor-2,3,6-trifluorbenzyloxy)-5-{3-[4-(2-hydroxymethylpyrrolidin-l-ylbutyl]ureido}-isothiazol-4-carbonsäureamid;
5-{3-[2-(1-Methylpyrrolidin-2-yl)ethyl]ureido}-3-(2,3,6-trifluor-4-methylbenzyloxy)isothiazol-4-carbonsäureamid;
5-[3-(4-Dimethylaminobutyl)ureido]-3-(2,3,6-trifluor-4-methylbenzyloxy)isothiazol-4-carbonsäureamid;
5-[3-(3-Dimethylaminopropyl)ureido]-3-(2,3,6-trifluor-4-methylbenzyloxy)isothiazol-4-carbonsäureamid;
5-[3-(3-Hydroxy-5-isopropropylaminopentyl)ureido]-3-(2,3,6-trifluor-4-methylbenzyloxy)isothiazol-4-carbonsäureamid;
5-[3-(3-Isopropylaminopropyl)ureido]-3-(2,3,6-trifluor-4-methylbenzyloxy)isothiazol-4-carbonsäureamid;
5-{3-[4-(4-Methylpiperazin-1-yl)butyl]ureido}-3-(2,3,6-trifluor-4-methylbenzyloxy)isothiazol-4-carbonsäureamid;
5-(3-{4-[4-(2-Hydroxyethyl)piperazin-1-yl]butyl}-ureido)-3-(2,3,6-trifluor-4-methylbenzyloxy)isothiazol-4-carbonsäureamid;
5-[3-(3-Pyrrolidin-1-ylpropyl)ureido]-3-(2,3,6-trifluor-4-methylbenzyloxy)isothiazol-4-carbonsäureamid;
5-[3-(4-Hydroxy-5-piperidin-1-ylpentyl)ureido]-3-(2,3,6-trifluor-4-methylbenzyloxy)isothiazol-4-carbonsäureamid;
3-(4-Chlor-2,6-difluorbenzyloxy)-5-[3-(4-imidazol-1-ylbutyl)ureido]isothiazol-4-carbonsäureamid;
5-(3-{4-[Ethyl(2-hydroxy-ethyl)amino]butyl}-ureido)-3-(2,3,6-trifluor-4-methylbenzyloxy)isothiazol-4-carbonsäureamid;
3-(4-Chlor-(2,3,6-trifluorbenzyloxy)-5-{3-[4-(2-hydroxmethylpiperidin-l-yl)butyl]ureido}isothiazol-4-carbonsäureamid;
3-(4-Chlor-2,3,6-trifluorbenzyloxy)-5-[3-(3-hydroxy-5-pyrrolidin-1-ylpentyl)ureido]isothiazol-4-carbonsäureamid;
3-(4-Brom-2,6-difluorbenzyloxy)-5-{3-[3-(4-methylpiperazin-1-yl)propyl]ureido}isothiazol-4-carbonsäureamid;
3-(2,6-Difluor-4-methylbenzyloxy)-5-{3-[2-(1-methylpyrrolidin-2-yl)ethyl]ureido}isothiazol-4-carbonsäureamid;
3-(2,6-Difluor-4-methylbenzyloxy)-5-[3-(4-dimethylaminobutyl)ureido}isothiazol-4-carbonsäureamid;
3-(2,6-Difluor-4-methylbenzyloxy)-5-[3-(3-dimethylaminopropyl)ureido]isothiazol-4-carbonsäureamid;
3-(4-Brom-2,3,6-trifluorbenzyloxy)-5-[3- (4-pyrrolidin-1-ylbutyl)ureido]isothiazol-4-carbonsäureamid;
3-(4-Chlor-2,3,6-trifluorbenzyloxy)-5-[3-(4-imidazol-1-ylbutyl)ureido]isothiazol-4-carbonsäureamid;
3-(4-Chlor-2,3,6-difluorbenzyloxy)-5-(3-{3-[ethyl-(2-hydroxyethyl)amino]propyl}ureido)isothiazol-4-carbonsäureamid;
3-(4-Chlor-2,3,6-trifluorbenzyloxy}-5-(3-{3-[ethyl(2-hydroxyethyl)amino]propyl}ureido)isothiazol-4-carbonsäureamid;
5-[3-(3-Methylaminopropyl)ureido]-3-(2,3,6-trifluor-4-methylbenzyloxy)isothiazol-4-carbonsäureamid;
5-[3-(3-Aminopropyl)-3-methylureido]-3-(2,3,6-trifluor-4-methylbenzyloxy)isothiazol-4-carbonsäureamid;
5-[3-(4-Diethylaminobutyl)ureido]-3-(2,3,6-trifluor-4-methylbenzyloxy)isothiazol-4-carbonsäureamid;
3-(2,6-Difluor-4-methylbenzyloxy)-5-[3-(3-pyrrolidin-1-ylpropyl)ureido]isothiazol-4-carbonsäureamid;
3-(3-Chlor-2,6-difluor-4-methylbenzyloxy)-5-[3-(4-dimethylaminobutyl)ureido]isothiazol-4-carbonsäureamid;
5-(3-{4-[Bis(2-hydroxyethyl)amino]butyl}ureido)-3-(2,6-difluor-4-methylbenzyloxy)isothiazol-4-carbonsäureamid;
und den pharmazeutisch unbedenklichen Salzen, Prodrugs und Solvaten der Verbindungen ausgewählt ist.

10. Verwendung nach Anspruch 1, wobei es sich bei der Verbindung der Formel 1 um das Hydrochloridsalz von 3-(4-Brom-2,6-difluorbenzyloxy)-5-[3-(4-pyrrolidin-1-ylbutyl)ureido]isothiazol-4-carbonsäureamid handelt.

11. Pharmazeutische Zusammensetzung zur Verwendung bei der Behandlung einer hyperproliferativen Störung bei einem Säugetier, die (i) Gemcitabin-hydrochlorid und (ii) eine therapeutisch wirksame Menge einer Verbindung der Formel 1 gemäß einem der Ansprüche 1 oder 6-10 in Kombination mit einem oder mehreren pharmazeutischen Trägern oder Vehikeln enthält.

12. Kit, das in einem ersten Kompartiment eine Verbindung der Formel 1 gemäß einem der Ansprüche 1 oder 6-10 und in einem zweiten Kompartiment Gemcitabin-hydrochlorid enthält.

13. Gemcitabin-hydrochlorid zur Verwendung bei der Behandlung von hyperproliferativen Störungen in Kombination mit einer Verbindung der Formel 1 gemäß einem der Ansprüche 1 oder 6-10 oder einem pharmazeutisch unbedenklichen Salz oder Solvat davon.

14. Verbindung der Formel 1 gemäß einem der Ansprüche 1 oder 6-10 oder ein pharmazeutisch unbedenkliches Salz oder Solvat davon zur Verwendung bei der Behandlung von hyperproliferativen Störungen in Kombination mit Gemcitabin-hydrochlorid.

15. Verbindung der Formel 1 gemäß einem der Ansprüche 1 oder 6-10 oder ein pharmazeutisch unbedenkliches Salz oder Solvat davon in Kombination mit Gemcitabin-hydrochlorid zur Verwendung bei der Behandlung von hyperproliferativen Störungen.

## Revendications

1. Utilisation de (i) une quantité thérapeutiquement efficace de chlorhydrate de gemcitabine et (ii) une quantité thérapeutiquement efficace d'un composé représenté par la formule 1 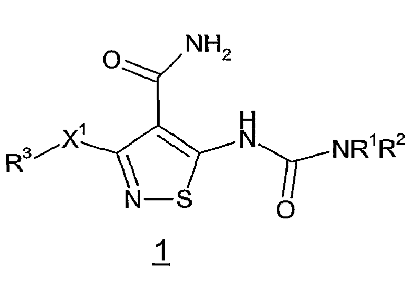 ou d'un sel ou solvate pharmaceutiquement acceptable de celui-ci, dans laquelle :
X¹ est O ou S ;
R¹ est H, un alkyle en C₁-C₁₀, un alcényle en C₂-C₁₀, un alcynyle en C₂-C₁₀, -C(O)(alkyle en C₁-C₁₀), -(CH₂)ₜ(aryle en C₆-C₁₀) , - (CH₂)ₜ(groupe hétérocyclique de 4-10 chaînons), -C(O)(CH₂)ₜ(aryle en C₆-C₁₀) ou -C(O)(CH₂)ₜ(groupe hétérocyclique de 5-10 chaînons), où t est un entier de 0 à 5 ; ledit groupe alkyle comprend facultativement 1 ou 2 hétérofractions choisies parmi 0, S et -N(R⁶)- à la condition que deux atomes 0, deux atomes S ou un atome O et un atome S ne soient pas attachés directement l'un à l'autre ; lesdits groupes R¹ aryles et hétérocycliques sont facultativement condensés à un groupe aryle en C₆-C₁₀, un groupe cyclique saturé en C₅-C₈ ou un groupe hétérocyclique de 5-10 chaînons ; 1 ou 2 atomes de carbone dans les fractions hétérocycliques susdites sont facultativement substitués par une fraction oxo (=O) ; les fractions -(CH2)ₜ- des groupes R¹ susdits comprennent facultativement une double ou triple liaison carbone-carbone quand t est un entier de 2 à 5 ; et les groupes R¹ susdits, sauf H, sont facultativement substitués par 1 à 3 groupes R⁴ ;
R² est choisi parmi la liste de substituants fournie dans la définition de R¹, -SO₂(CH₂)ₜ(aryle en C₆-C₁₀), -SO₂(CH₂)ₜ(groupe hétérocyclique de 5-10 chaînons) et -OR⁵, t est un entier allant de 0 à 5, les fractions -(CH₂)ₜ- des groupes R² susdits comprennent facultativement une double ou triple liaison carbone-carbone quand t est un entier de 2 à 5 et les groupes R² susdits sont facultativement substitués par 1 à 3 groupes R⁴;
ou R¹ et R² peuvent être pris conjointement avec l'azote auquel ils sont attachés pour former un noyau saturé monocyclique ou polycyclique de 4-10 chaînons ou un noyau hétéroaryle de 5-10 chaînons, où lesdits noyaux saturé et hétéroaryle comprennent facultativement 1 ou 2 hétéroatomes choisis parmi 0, S et -N(R⁶)- en plus de l'azote auquel R¹ et R² sont attachés, ledit -N(R⁶) - est facultativement =N- ou -N= quand R¹ et R² sont pris ensemble sous forme dudit groupe hétéroaryle, ledit noyau saturé peut facultativement être partiellement insaturé par inclusion de 1 ou 2 doubles liaisons carbone-carbone et lesdits noyaux saturé et hétéroaryle, y compris le groupe R⁶ dudit -N(R⁶)-, sont facultativement substitués par 1 à 3 groupes R⁴ ;
R³ est H, un alkyle en C₁-C₁₀, un alcényle en C₂-C₁₀, un alcynyle en C₂-C₁₀, -(CH₂)ₜ(aryle en C₆-C₁₀) ou -(CH₂)ₜ(groupe hétérocyclique de 5-10 chaînons), où t est un entier de 0 à 5 ; ledit groupe alkyle comprend facultativement 1 ou 2 hétérofractions choisies parmi 0, S et -N(R⁶)- à la condition que deux atomes 0, deux atomes S ou un atome 0 et un atome S ne soient pas attachés directement l'un à l'autre ; lesdits groupes R³ aryles et hétérocycliques sont facultativement condensés à un groupe aryle en C₆-C₁₀, un groupe cyclique saturé en C₅-C₈ ou un groupe hétérocyclique de 5-10 chaînons ; 1 ou 2 atomes de carbone dans les fractions hétérocycliques susdites sont facultativement substitués par une fraction oxo (=O) ; les fractions -(CH₂)ₜ- des groupes R³ susdits comprennent facultativement une double ou triple liaison carbone-carbone quand t est un entier de 2 à 5 ; et les groupes R³ susdits sont facultativement substitués par 1 à 5 groupes R⁴ ;
chaque R⁴ est indépendamment choisi parmi un alkyle en C₁-C₁₀, un alcényle en C₂-C₁₀, un alcynyle en C₂-C₁₀, un halogéno, un cyano, un nitro, un trifluorométhyle, un trifluorométhoxy, un azido, -OR⁵, -C(O)R⁵ -C(O)OR⁵, -NR⁶C(O)OR⁵, -OC(O)R⁵, -NR⁶SO₂R⁵, -SO₂NR⁵R⁶, -NR⁶C(O)R⁵, -C(O)NR⁵R⁶, NR⁵R⁶, -S(O)ⱼR⁷ où j est un entier allant de 0 à 2, -SO₃H, -NR⁵(CR⁶R⁷)tOR⁶, - (CH2)t(aryle en C₆-C₁₀) , -SO₂(CH₂)ₜ(aryle en C₆-C₁₀) , -S(CH₂)ₜ(aryle en C₆-C₁₀), -O(CH₂)ₜ(aryle en C₆-C₁₀), -(CH₂)ₜ(groupe hétérocyclique de 5-10 chaînons) et -(CR⁶R⁷)ₘOR⁶, où m est un entier de 1 à 5 et t est un entier de 0 à 5 ; ledit groupe alkyle contient facultativement 1 ou 2 hétérofractions choisies parmi 0, S et -N(R⁶)- à la condition que deux atomes 0, deux atomes S ou un atome 0 et un atome S ne soient pas attachés directement l'un à l'autre ; lesdits groupes R⁴ aryles et hétérocycliques sont facultativement condensés à un groupe aryle en C₆-C₁₀, un groupe cyclique saturé en C₅-C₆ ou un groupe hétérocyclique de 5-10 chaînons ; 1 ou 2 atomes de carbone dans les fractions hétérocycliques susdites sont facultativement substitués par une fraction oxo (=O) ; et les fractions alkyles, aryles et hétérocycliques des groupes R⁴ susdits sont facultativement substituées par 1 à 3 substituants indépendamment choisis parmi un halogéno, un cyano, un nitro, un trifluorométhyle, un trifluorométhoxy, un azido, -NR⁶SO₂R⁵, -SO₂NR⁵R⁶, -C(O)R⁵, -C(O)OR⁵, -OC(O)R⁵, -NR⁶C(O)R⁵, -C(O)NR⁵R⁶, -NR⁵R⁶, -(CR⁶R⁷)ₘOR⁶ où m est un entier de 1 à 5, -OR⁵ et les substituants listés dans la définition de R⁵ ;
chaque R⁵ est indépendamment choisi parmi H, un alkyle en C₁-C₁₀, -(CH₂)ₜ(aryle en C₆-C₁₀) et -(CH₂)ₜ(groupe hétérocyclique de 5-10 chaînons), où t est un entier de 0 à 5 ; ledit groupe alkyle comprend facultativement 1 ou 2 hétérofractions choisies parmi 0, S et -N(R⁶)- à la condition que deux atomes 0, deux atomes S ou un atome O et un atome S ne soient pas attachés directement l'un à l'autre ; lesdits groupes R⁵ aryles et hétérocycliques sont facultativement condensés à un groupe aryle en C₆-C₁₀, un groupe cyclique saturé en C₅-C₈ ou un groupe hétérocyclique de 5-10 chaînons ; et les substituants R⁵ susdits, sauf H, sont facultativement substitués par 1 à 3 substituants indépendamment choisis parmi un halogéno, un cyano, un nitro, un trifluorométhyle, un trifluorométhoxy, un azido, -C(O)R⁶, -C(O)OR⁶ -CO(O)R⁶, -NR⁶C(O)R⁷, -C(O)NR⁶R⁷, NR⁶R⁷, un hydroxy, un alkyle en C₁-C₆ et un alcoxy en C₁-C₆ ; et
chaque R⁶ et R⁷ est indépendamment H ou un alkyle en C₁-C₆ ;
pour la fabrication de produits pharmaceutiques pour administration simultanée ou séquentielle pour le traitement de troubles hyperprolifératifs.

2. Utilisation selon la revendication 1, dans laquelle le trouble hyperprolifératif est un cancer.

3. Utilisation selon la revendication 2, dans laquelle ledit cancer est choisi dans le groupe constitué par le cancer du cerveau, malpighien, de la vessie, de l'estomac, du pancréas, du sein, de la tête, du cou, de l'oesophage, de la prostate, colorectal, du poumon, du rein, de l'ovaire, gynécologique et de la thyroïde.

4. Utilisation sel o n l'une quelconque des revendications 1 à 3, dans laquelle lesdits composés (i) et (ii) sont administrés simultanément.

5. Utilisation selon l'une quelconque des revendications 1 à 3, dans laquelle lesdits composés (i) et (ii) sont administrés séquentiellement.

6. Utilisation selon l'une quelconque des revendications 1 à 5, dans laquelle R² du composé de formule 1 est H et R¹ est un alkyle en C₁-C₁₀ facultativement substitué par 1 ou 2 substituants indépendamment choisis parmi -NR⁵R⁶, -NR⁵(CR⁶R⁷)ₜOR⁶ et -(CH₂)ₜ(groupe hétérocyclique de 5-10 chaînons) où t est un entier de 0 à 5.

7. Utilisation selon l'une quelconque des revendications 1 à 5, dans laquelle R² du composé de formule 1 est H et R¹ du composé de formule 1 est -(CH₂)ₜ(groupe hétérocyclique de 5-10 chaînons), où t est un entier de 0 à 5 ; ledit groupe hétérocyclique est facultativement condensé à un groupe aryle en C₆-C₁₀, un groupe cyclique saturé en C₅-C₈ ou un groupe hétérocyclique de 5-10 chaînons ; et ledit groupe R¹, y compris les portions facultativement condensées dudit groupe R¹, est facultativement substitué par 1 ou 2 substituants indépendamment choisis parmi un alkyle en C₁-C₄, un hydroxy et un hydroxyméthyle.

8. Utilisation selon l'une quelconque des revendications 1 à 5, dans laquelle R³ du composé de formule 1 est -(CH₂)ₜ(aryle en C₆-C₁₀) où t est un entier de 1 à 3 et ledit groupe R³ est facultativement substitué par 1 à 4 groupes R⁴.

9. Utilisation selon l'une quelconque des revendications 1 à 5, dans laquelle le composé de formule 1 est choisi dans le groupe constitué par le sel de mésylate de l'amide de l'acide 3-(4-bromo-2,6-difluorobenzyloxy)-5-[3-(4-pyrrolidin-1-ylbutyl)uréido]isothiazole-4-carboxylique ;
l'amide de l'acide 5-{3-[3-(4-méthylpipérazin-1-yl)propyl]uréido}-3-(2,3,6-trifluoro-4-méthylbenzyloxy)isothiazole-4-carboxylique ;
l'amide de l'acide 3-(4-chloro-2,6-difluorobenzyloxy)-5-(3-{4-[éthyl(2-hydroxyéthyl)amino]butyl}uréido)isothiazole-4-carboxylique ;
l'amide de l'acide 3-(2-fluoro-4-méthylbenzyloxy)-5-{3-[3-(4-méthylpipérazin-1-yl)propyl]uréido}isothiazole-4-carboxylique ;
l'amide de l'acide 3-(2,5-difluoro-4-méthylbenzyloxy)-5-(3-{4-[4-(2-hydroxyéthyl)pipérazin-1-yl]butyl}uréido)isothiazole-4-carboxylique ;
l'amide de l'acide 3-(2,5-difluoro-4-méthylbenzyloxy)-5-[3-(6-diméthylaminohexyl)uréido]isothiazole-4-carboxylique ;
l'amide de l'acide 3-(2-fluoro-4-méthylbenzyloxy)-5-[3-(5-isopropylaminopentyl)uréido]isothiazole-4-carboxylique ;
le sel de chlorhydrate de l'amide de l'acide 3-(4-bromo-2,6-difluorobenzyloxy)-5-[3-(4-pyrrolidin-1-ylbutyl)uréido]isothiazole-4-carboxylique ;
l'amide de l'acide 3-(4-chloro-2,6-difluorobenzyloxy)-5-[3-(4-pyrrolidin-1-ylbutyl)uréido]isothiazole-4-carboxylique ;
l'amide de l'acide 3-(4-chloro-2,6-difluorobenzyloxy)-5-{3-[3-(4-méthylpipérazin-1-yl)propyl]uréido}isothiazole-4-carboxylique ;
l'amide de l'acide 3-(4-chloro-2,6-difluorobenzyloxy)-5-{3-[-(1-méthylpyrrolidin-2-yl)éthyl]uréido}isothiazole-4-carboxylique ;
l'amide de l'acide 3-(2,6-difluoro-4-méthylbenzyloxy)-5-[3-(4-pyrrolidin-1-ylbutyl)uréido]isothiazole-4-carboxylique ;
l'amide de l'acide 3-(2,6-difluoro-4-méthylbenzyloxy)-5-(3-{4-[4-(2-hydroxyéthyl)pipérazin-1-yl]butyl}uréido)isothiazole-4-carboxylique ;
l'amide de l'acide 3-(4-chloro-2,6-difluorobenzyloxy)-5-[3-(3-hydroxy-5-pyrrolidin-1-yl)pentyl)uréido]isothiazole-4-carboxylique ;
l'amide de l'acide 3-(2,5-difluoro-4-méthylbenzyloxy)-5-{3-[4-(3,4-dihydroxypyrrolidin-1-yl)butyl]uréido}isothiazole-4-carboxylique ;
l'amide de l'acide 3-(4-chloro-2,6-difluorobenzyloxy)-5-{3-[4-(3,4-dihydroxypyrrolidin-1-yl)butyl]uréido}isothiazole-4-carboxylique ;
l'amide de l'acide 3-(2,5-difluoro-4-méthylbenzyloxy)-5-{3-[4-(2-hydroxyméthylpyrrolidin-1-yl)butyl]uréido}isothiazole-4-carboxylique ;
l'amide de l'acide 3-(4-chloro-2,6-difluorobenzyloxy)-5-{3-[4-(2-hydroxyméthylpyrrolidin-1-yl)butyl]uréido}isothiazole-4-carboxylique ;
l'amide de l'acide 3-(2,5-difluoro-4-méthylbenzyloxy)-5-{3-[4-(3-hydroxypyrrolidin-1-yl)butyl]uréido}isothiazole-4-carboxylique ;
l'amide de l'acide 3-(4-bromo-2,6-difluorobenzyloxy)-5-[3-(4-pyrrolidin-1-ylbutyl)uréido]isothiazole-4-carboxylique ;
l'amide de l'acide 3-(2,6-difluoro-4-méthylbenzyloxy)-5-[3-(4-hydroxy-5-pipéridin-1-ylpentyl)uréido]isothiazole-4-carboxylique ;
l'amide de l'acide 3-(2,5-difluoro-4-méthylbenzyloxy)-5-{3-[4-(3-hydroxy-5-pipéridin-1-ylpentyl)uréido]isothiazole-4-carboxylique ;
l'amide de l'acide 3-(4-chloro-2,6-difluorobenzyloxy)-5-{3-[4-(2-hydroxyméthylpipéridin-1-yl)butyl]uréido}isothiazole-4-carboxylique ;
l'amide de l'acide 3-(2,5-difluoro-4-méthylbenzyloxy)-5-(3-{4-[éthyl(2-hydroxyéthyl)amino]butyl}uréido)isothiazole-4-carboxylique ;
l'amide de l'acide 3-(4-chloro-2,6-difluorobenzyloxy)-5-[3-(5-hydroxy-6-pipéridin-1-yl)hexyl)uréido}isothiazole-4-carboxylique ;
l'amide de l'acide 3-(4-bromo-2,3,6-trifluorobenzyloxy)-5-{3-[3-(4-méthylpipérazin-1-yl)propyl]uréido}isothiazole-4-carboxylique ;
l'amide de l'acide 3-(2,6-difluoro-4-méthylbenzyloxy)-5-{3-[3-(4-méthylpipérazin-1-yl)propyl]uréido}isothiazole-4-carboxylique ;
l'amide de l'acide 3-(2,6-difluoro-4-méthylbenzyloxy)-5-[3-(3-hydroxy-5-pyrrolidin-1-ylpentyl)uréido]isothiazole-4-carboxylique ;
l'amide de l'acide 5-[3-(4-pyrrolidin-1-ylbutyl)uréido]-3-(2,3,6-trifluoro-4-méthylbenzyloxy)isothiazole-4-carboxylique ;
l'amide de l'acide 5-[3-(3-hydroxy-5-pyrrolidin-1-ylpentyl)uréido]-3-(2,3,6-trifluoro-4-méthylbenzyloxy)isothiazole-4-carboxylique ;
l'amide de l'acide 3-(4-chloro-2,6-difluorobenzyloxy)-5-{3-[3-(5-méthyl-2,5-diazabicyclo[2.2.1]hept-2-yl)propyl]uréido}isothiazole-4-carboxylique ;
l'amide de l'acide 3-(4-chloro-2,3,6-trifluorobenzyloxy)-5-{3-[3-(5-méthyl-2,5-diazabicyclo[2.2.1]hept-2-yl)propyl]uréido}isothiazole-4-carboxylique ;
l'amide de l'acide 3-(4-chloro-2,3,6-trifluorobenzyloxy)-5-{3-[2-(1-méthylpyrrolidin-2-yl)éthyl]uréido}isothiazole-4-carboxylique ;
l'amide de l'acide 3-(4-chloro-2,3,6-trifluorobenzyloxy)-5-[3-(4-pyrrolidin-1-ylbutyl)uréido]isothiazole-4-carboxylique ;
l'amide de l'acide 3-(4-chloro-2,3,6-trifluorobenzyloxy)-5-{3-[4-(2-hydroxyméthylpyrrolidin-1-yl)butyl]uréido}isothiazole-4-carboxylique ;
l'amide de l' acide 5-{3-[2-(1-méthylpyrrolidin-2-yl)éthyl]uréido}-3-(2,3,6-trifluoro-4-méthylbenzyloxy)isothiazole-4-carboxylique ;
l'amide de l'acide 5-[3-(4-diméthylaminobutyl)uréido]-3-(2,3,6-trifluoro-4-méthylbenzyloxy)isothiazole-4-carboxylique ;
l'amide de l'acide 5-[3-(3-diméthylaminopropyl)uréido]-3-(2,3,6-trifluoro-4-méthylbenzyloxy)isothiazole-4-carboxylique ;
l'amide de l'acide 5-[3-(3-hydroxy-5-isopropropylaminopentyl)uréido]-3-(2,3,6-trifluoro-4-méthylbenzyloxy)isothiazole-4-carboxylique ;
l'amide de l'acide 5-[3-(3-isopropylaminopropyl)uréido]-3-(2,3,6-trifluoro-4-méthylbenzyloxy)isothiazole-4-carboxylique ;
l'amide de l'acide 5-{3-[4-(4-méthylpipérazin-1-yl)butyl]uréido}-3-(2,3,6-trifluoro-4-méthylbenzyloxy)isothiazole-4-carboxylique ;
l'amide de l'acide 5-(3-{4-[4-(2-hydroxyéthyl)pipérazin-1-yl]butyl}uréido)-3-(2,3,6-trifluoro-4-méthylbenzyloxy)isothiazole-4-carboxylique ;
l'amide de l'acide 5-[3-(3-pyrrolidin-1-ylpropyl)uréido]-3-(2,3,6-trifluoro-4-méthylbenzyloxy)isothiazole-4-carboxylique ;
l'amide de l'acide 5-[3-(4-hydroxy-5-pipéridin-1-ylpentyl)uréido]-3-(2,3,6-trifluoro-4-méthylbenzyloxy)isothiazole-4-carboxylique ;
l'amide de l'acide 3-(4-chloro-2,6-difluorobenzyloxy)-5-[3-(4-imidazol-1-ylbutyl)uréido]isothiazole-4-carboxylique ;
l'amide de l'acide 5-(3-{4-[éthyl(2-hydroxyéthyl)amino]butyl}uréido)-3-(2,3,6-trifluoro-4-méthylbenzyloxy)isothiazole-4-carboxylique ;
l'amide de l'acide 3-(4-chloro-2,3,6-trifluorobenzyloxy)-5-{3-[4-(2-hydroxyméthylpipéridin-1-yl)butyl]uréido}isothiazole-4-carboxylique ;
l'amide de l'acide 3-(4-chloro-2,3,6-trifluorobenzyloxy)-5-[3-(3-hydroxy-5-pyrrolidin-1-ylpentyl)uréido]isothiazole-4-carboxylique ;
l'amide de l'acide 3-(4-bromo-2,6-difluorobenzyloxy)-5-{3-[3-(4-méthylpipérazin-1-yl)-propyl]uréido}isothiazole-4-carboxylique ;
l'amide de l'acide 3-(2,6-difluoro-4-méthylbenzyloxy)-5-{3-[2-(1-méthylpyrrolidin-2-yl)éthyl]uréido}isothiazole-4-carboxylique ;
l'amide de l'acide 3-(2,6-difluoro-4-méthylbenzyloxy)-5-[3-(4-diméthylaminobutyl)uréido]isothiazole-4-carboxylique ;
l'amide de l'acide 3-(2,6-difluoro-4-méthylbenzyloxy)-5-[3-(3-diméthylaminopropyl)uréido]isothiazole-4-carboxylique ;
l'amide de l'acide 3-(4-bromo-2,3,6-trifluorobenzyloxy)-5-[3-(4-pyrrolidin-1-ylbutyl)uréido]isothiazole-4-carboxylique ;
l'amide de l'acide 3-(4-chloro-2,3,6-trifluorobenzyloxy)-5-[3-(4-imidazol-1-ylbutyl)uréido]isothiazole-4-carboxylique ;
l'amide de l'acide 3-(4-chloro-2,3,6-difluorobenzyloxy)-5-(3-{3-[éthyl(2-hydroxyéthyl)amino]propyl}uréido)isothiazole-4-carboxylique ;
l'amide de l'acide 3-(4-chloro-2,3,6-trifluorobenzyloxy)-5-(3-{3-[éthyl(2-hydroxyéthyl)amino]propyl}uréido)isothiazole-4-carboxylique ;
l'amide de l'acide 5-[3-(3-méthylaminopropyl)uréido]-3-(2,3,6-trifluoro-4-méthylbenzyloxy)isothiazole-4-carboxylique ;
l'amide de l'acide 5-[3-(3-aminopropyl)-3-méthyluréido]-3-(2,3,6-trifluoro-4-méthylbenzyloxy)isothiazole-4-carboxylique ;
l'amide de l'acide 5-[3-(4-diéthylaminobutyl)uréido]-3-(2,3,6-trifluoro-4-méthylbenzyloxy)isothiazole-4-carboxylique ;
l'amide de l'acide 3-(2,6-difluoro-4-méthylbenzyloxy)-5-[3-(3-pyrrolidin-1-ylpropyl)uréido]isothiazole-4-carboxylique ;
l'amide de l'acide 3-(3-chloro-2,6-difluoro-4-méthylbenzyloxy)-5-[3-(4-diméthylaminobutyl)uréido]isothiazole-4-carboxylique ;
l'amide de l'acide 5-(3-{4-[bis(2-hydroxyéthyl)amino]butyl}uréido)-3-(2,6-difluoro-4-méthylbenzyloxy)isothiazole-4-carboxylique ;
et les sels, promédicaments et solvates pharmaceutiquement acceptables desdits composés.

10. Utilisation selon la revendication 1, dans laquelle le composé de formule 1 est le sel de chlorhydrate de l'amide de l'acide 3-(4-bromo-2,6-difluorobenzyloxy)-5-[3-(4-pyrrolidin-1-ylbutyl)uréido]isothiazole-4-carboxylique.

11. Composition pharmaceutique destinée à être utilisée dans le traitement d'un trouble hyperprolifératif chez un mammifère laquelle comprend (i) du chlorhydrate de gemcitabine et (ii) une quantité thérapeutiquement efficace d'un composé représenté par la formule 1, tel que défini dans l'une quelconque des revendications 1 ou 6-10, en association avec un ou plusieurs vecteurs ou véhicules pharmaceutiques.

12. Trousse comprenant dans un premier compartiment un composé de formule 1 tel que défini dans l'une quelconque des revendications 1 ou 6-10 et dans un second compartiment du chlorhydrate de gemcitabine.

13. Chlorhydrate de gemcitabine destiné à être utilisé dans le traitement de troubles hyperprolifératifs en association avec un composé de formule 1, tel que défini dans l'une quelconque des revendications 1 ou 6-10, ou un sel ou solvate pharmaceutiquement acceptable de celui-ci.

14. Composé de formule 1, tel que défini dans l'une quelconque des revendications 1 ou 6-10, ou sel ou solvate pharmaceutiquement acceptable de celui-ci destiné à être utilisé dans le traitement de troubles hyperprolifératifs en association avec du chlorhydrate de gemcitabine.

15. Composé de formule 1, tel que défini dans l'une quelconque des revendications 1 ou 6-10, ou sel ou solvate pharmaceutiquement acceptable de celui-ci en association avec du chlorhydrate de gemcitabine destiné à être utilisé dans le traitement de troubles hyperprolifératifs.
